(19) Europäisches Patentamt
European Patent Office
Office européen des brevets

(11) **EP 4 445 949 A1**

(12) **EUROPEAN PATENT APPLICATION**
published in accordance with Art. 153(4) EPC


(43) Date of publication:
**16.10.2024 Bulletin 2024/42**

(21) Application number: **22904177.7**

(22) Date of filing: **05.12.2022**

(51) International Patent Classification (IPC):
***A61P 1/00*** *(2006.01)* ***A61P 1/16*** *(2006.01)*
***A61P 43/00*** *(2006.01)* ***C12N 15/09*** *(2006.01)*
***A61K 31/14*** *(2006.01)* ***A61K 31/198*** *(2006.01)*
***A61K 31/205*** *(2006.01)*

(52) Cooperative Patent Classification (CPC):
**A61K 31/14; A61K 31/198; A61K 31/205;**
**A61P 1/00; A61P 1/16; A61P 43/00; C12N 15/09**

(86) International application number:
**PCT/JP2022/044650**

(87) International publication number:
**WO 2023/106248 (15.06.2023 Gazette 2023/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.12.2021 JP 2021197450**



(71) Applicant: **The University of Tokyo**
**Bunkyo-ku, Tokyo 113-8654 (JP)**

(72) Inventors:
- **HAYASHI Hisamitsu**
**Tokyo 113-8654 (JP)**
- **SABU Yusuke**
**Tokyo 113-8654 (JP)**
- **TAMURA Ryutaro**
**Tokyo 113-8654 (JP)**

(74) Representative: **Vossius & Partner**
**Patentanwälte Rechtsanwälte mbB**
**Siebertstrasse 3**
**81675 München (DE)**


Remarks:
The complete document including Reference Table(s) and the Sequence Listing(s) can be downloaded from the EPO website

# (54) MEDICINE FOR STEATOHEPATITIS


(57) It is an object of the present invention to provide a preventive agent, a therapeutic agent or a therapeutic composition for steatohepatitis developed by phospholipid deficiency or decreased phospholipid synthesis in the liver, and to provide a therapeutic method for the aforementioned disease. More specifically, the present invention relates to a therapeutic agent for steatohepatitis, comprising, as an active ingredient, choline or a choline metabolite, ethanolamine or an ethanolamine metabolite, a salt thereof, or a solvate thereof, wherein the therapeutic agent for steatohepatitis is characterized in that the steatohepatitis is developed by decreased phospholipid synthesis in the liver. Examples of the choline metabolite may include betaine, dimethylglycine, and phosphatidylcholine.


[Figure 8]

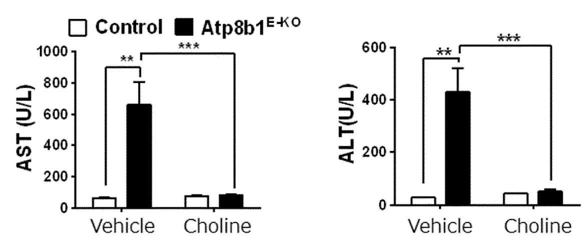


EP 4 445 949 A1

## Description

### Technical Field

The present invention relates to a therapeutic agent or a therapeutic composition for diseases caused by dysfunction of ATP8B 1, such as, for example, progressive familial intrahepatic cholestasis type 1.

### Background Art

The condition, in which the secreted amount of bile substances is decreased and bile components leak into the blood, is called cholestasis. Progressive familial intrahepatic cholestasis (PFIC) is a disease that congenitally causes intrahepatic cholestasis. PFIC is divided into five subtypes, PFIC1-5, according to the causative genes. In progressive familial intrahepatic cholestasis type 1 (PFIC1), ATP8B1 encoding ATP8B 1, which acts as a flippase for aminophospholipids in the cell membrane, has been reported as a causative gene (Non Patent Literatures 1 and 2). The flippase is a protein that transfers phospholipids from the outside to the inside of the cell membrane. In general, lipid molecules can move relatively freely within the layer in which they are present, but they cannot cross between bilayers by themselves, and therefore, flippase-mediated transportation of phospholipids is considered to contribute to the asymmetry of the bilayer.

At present, the following two hypotheses have been proposed for the pathogenic onset mechanism of PFIC1. One is the hypothesis involving a decline in the function of the causative gene BSEP of PFIC2. It has been reported that phosphatidylserine, which is not normally comprised in bile, is detected in ATP8B1 function-deficient mice, and the amount of cholesterol in bile is large, and that a reduction in the cholesterol content in the cell membrane causes a decline in the function of BSEP. Thus, the first hypothesis is that a loss of ATP8B1 function indirectly reduces the function of BSEP and provokes intrahepatic cholestasis (Non Patent Literature 3). The second hypothesis is that a loss of ATP8B 1 function causes down-regulation of FXR (farnesoid X receptor) and suppression of nuclear translocation, resulting in a decrease in the expression of BSEP expression due to gene regulation downstream of FXR (Non Patent Literature 4). In both hypotheses, choleretic action through the enhancement of BSEP function is expected to bring on therapeutic effects on the present disease. However, sodium phenylbutyrate, a BSEP enhancer found by the present inventors, did not exhibit therapeutic effects on PFIC1, to such an extent that extension of autologous liver survival was expected (Non Patent Literature 5). Based on these results, the above-described two hypotheses are considered to be denied.

As mentioned above, elucidation of the physiological function of ATP8B1 is insufficient, and the role of ATP8B1 in the pathologic onset of PFIC1 is still unknown.

### Citation List

### Non Patent Literature

Non Patent Literature 1: Sambrotta et al., Nat. Genet., 46: 326-328, 2014, doi: 10.1038/ng.2918.

Non Patent Literature 2: Gomez-Ospina et al., Nat. Commun., 7: 10713, 2014, doi: 10.1038/ncomms10713.

Non Patent Literature 3: Folmer et al., Biochim. Biophys. Acta - Mol. Cell Biol. Lipids, 1791: 628-635, 2009, doi: 10.1016/j.bbalip.2009.02.008.

Non Patent Literature 4: Frankenberg et al., Hepatology, 48: 1896-1905, 2008, doi: 10.1002/hep.22431.

Non Patent Literature 5: Hasegawa et al., Orphanet J. Rare Dis., 9: 1-9, 2014, doi: 10.1186/1750-1172-9-89.


### Summary of Invention

### Technical Problem

Considering the aforementioned circumstances, it is an object of the present invention to provide a therapeutic agent or a therapeutic composition for diseases developed by ATP8B1 function decline in intestinal epithelial cells, such as, for example, progressive familial intrahepatic cholestasis type 1 (PFIC1), and also to provide a method for treating the aforementioned diseases.

In addition, it is another object of the present invention to provide a medicament or a pharmaceutical composition for prevention or treatment of steatohepatitis developed by decreased phospholipid synthesis in the liver, such as progressive familial intrahepatic cholestasis type 1 (PFIC1), and a preventive method and a therapeutic method for the aforementioned disease.

Solution to Problem

To date, the present inventors have produced intestinal epithelial cell-specific Atp8b 1 knockout mice and have investigated the role of Atp8b 1 in the pathogenic onset of PFIC1.

The present inventors have found that the intestinal epithelial cell-specific Atp8b1 knockout (Atp8b1 E-KO) mice have a mortality rate of approximately 70% by 8 weeks of age, and exhibit findings such as growth defects, an increase in liver injury markers, and a decrease in secondary bile acids, which are consistent with cholestasis liver injury. In addition, the histological analysis of the liver showed mild cholestasis. Moreover, the Atp8b 1 E-KO mice exhibited abnormal intestinal morphology. Based on the aforementioned findings, it is considered that the Atp8b1 E-KO mice can be utilized as PFIC1 mouse models.

Furthermore, the present inventors have performed lipidome analysis using the intestinal epithelial cells of Atp8b1 E-KO mice. As a result, the inventors have found that lysophospholipids accumulated in the intestinal epithelial cells of the Atp8b1 E-KO mice. From these results, it has been suggested that Atp8b1 is likely to act as a flippase of the lysophospholipids from the outer membrane to the inner membrane in the luminal membrane of the intestinal epithelium. In other words, it has been suggested that when ATP8B1 in intestinal epithelial cells undergoes some functional decline, the flippase activity of ATP8B1 is decreased, and as a result, lysophospholipids such as lysophosphatidylcholine (LPC) and lysophosphatidylethanolamine (LPE) do not move from the outer membrane to the inner membrane of intestinal epithelial cells, so that the uptake of the lysophospholipids into the intestinal epithelial cells is inhibited.

Normally, after LPC has been absorbed in intestinal epithelial cells, most of it is converted to phosphatidylcholine (PC) in the intestinal epithelial cells. Thereafter, PC is transported to the liver via blood in the portal vein, and choline is then synthesized from PC in the liver. Such choline is also utilized for the biosynthesis of PC in the liver. In the case of LPE, after LPE has been absorbed in intestinal epithelial cells, it is converted to phosphatidylethanolamine (PE) or ethanolamine in the intestinal epithelial cells. PE and ethanolamine are transported to the liver via blood in the portal vein and are then converted to PC in the liver (Biochim Biophys Acta Biomembr., 1859: 1558-1575, 2017). Also from PC derived from LPE, choline is synthesized in the liver (see Figure 2).

From the aforementioned results, the present inventors have assumed that when some dysfunction (e.g., function decline) occurs in ATP8B 1 in intestinal epithelial cells, in addition to a decrease in the supply of phospholipids into the liver, phospholipid raw materials such as choline and ethanolamine are depleted in the liver, leading to a decrease in the synthesis of phospholipids.

In fact, choline concentrations in the blood and liver of Atp8b1 E-KO mice were reduced by approximately 50%. In addition, in the liver histopathological analysis of Atp8b1 E-KO mice, a significant accumulation of lipid droplets was observed, compared with control mice, and in biochemical tests, an increase in triglyceride (TG) concentration in the liver was confirmed. On the other hand, the TG concentration in the blood was significantly decreased in Atp8b1 E-KO mice, compared with control mice, suggesting that very low-density lipoprotein (VLDL) synthesis was decreased due to intrahepatic choline deficiency.

These findings suggest that disruption of Atp8b 1 function in intestinal epithelial cells induces not only a decrease in the supply of phospholipids into the liver, but also abnormal phospholipid biosynthesis via deficiency of choline, ethanolamine, and their metabolites in the liver, and as a result, it may lead to various pathological conditions, such as fatty liver and fatty liver-associated cholestasis. Thus, the present inventors have assumed that a choline-added diet would be effective for preventing or improving diseases or pathological conditions caused by phospholipid deficiency and abnormal phospholipid biosynthesis in the liver, which are generated by ATP8B1 function decline in intestinal epithelial cells or other causes, such as, for example, PFIC1, short bowel syndrome and inflammatory bowel disease, and the inventors have conducted further investigation.

The present inventors fed mothers of Atp8b1 E-KO mice with a 1% choline supplemented diet, and found that the liver injury markers and pathological findings in Atp8b1 E-KO pups were normalized. Furthermore, the inventors examined the abundance of choline and metabolites thereof in the plasma and liver of the Atp8b1 E-KO pups. As a result, it was unexpectedly found that there was no change in the amount of choline in the pups between the case where the mother mice had been fed with a normal diet and the case where the mother mice had been fed with a 1% choline supplemented diet. However, it was found that the amounts of choline metabolites, namely, betaine, dimethylglycine, and phosphatidylcholine (PC), were increased when the mother mice were fed with a 1% choline supplemented diet, indicating that the amount of choline was recovered up to the same level as that in the control mice (wild-type mice).

Furthermore, taking into consideration that ATP8B1 is responsible for the supply of phospholipids (PC and PE) into the liver and also for the supply of raw materials (choline and ethanolamine) necessary for phospholipid biosynthesis in the liver, via its function of absorbing LPC and LPE in the epithelial cells of the lower part of small intestine (see Figure 16), the present inventors have assumed that choline or choline metabolites are likely to exhibit therapeutic effects also on short bowel syndrome in which the lower part of the small intestine is resected (Radetic et al., Dig Dis Sci. 2022 Nov 25. doi: 10.1007/s10620-022-07760-w.), and inflammatory bowel disease in which the lower part of the small intestine becomes dysfunctional (Roberts et al., J Crohns Colitis. 2020 Sep 7; 14(8): 1119-1148. doi: 10.1093/ecco jcc/jjaa037;

Forsdick et al., J Pediatr Surg. 2019 Dec; 54(12): 2554-2558. doi: 10.1016/j.jpedsurg.2019.08.036. Epub 2019 Oct 15.; Zou et al., Inflamm Bowel Dis. 2019 Oct 18; 25(11): 1764-1772. doi: 10.1093/ibd/izz043.), and the present inventors have proceeded with the analyses. With regard to short bowel syndrome, inflammatory bowel disease, and pediatric obesity, which are associated with the onset of steatohepatitis, as with PFIC1, the concentrations of choline and betaine in blood were measured. As a result, it was confirmed that the concentrations of choline and betaine in blood were decreased in subjects with short bowel syndrome, inflammatory bowel disease, and pediatric obesity, compared with those of healthy children.

From these results, it has been suggested that choline or choline metabolites exhibit effects on the prevention and improvement of steatohepatitis developed by phospholipid deficiency or decreased phospholipid synthesis in the liver, which is generated by ATP8B1 function decline or other causes. Furthermore, taking into consideration that ethanolamine is converted to PC in the liver (Biochim Biophys Acta Biomembr., 1859:1558-1575 2017), and that choline is synthesized from PC in the liver (see Figure 2), these results strongly suggest that, as with choline, ethanolamine (and a metabolite thereof), a main raw material for phospholipid biosynthesis in the liver, exhibit effects on the prevention or improvement of steatohepatitis developed by phospholipid deficiency or decreased phospholipid synthesis in the liver.

The present invention was completed based on the above-described findings.

Specifically, the present invention includes the following (1) to (10).

(1) A medicament for steatohepatitis, comprising, as an active ingredient, choline or a choline metabolite, ethanolamine or an ethanolamine metabolite, a salt thereof, or a solvate thereof, wherein the medicament for steatohepatitis is characterized in that steatohepatitis is developed by phospholipid deficiency or decreased phospholipid synthesis in the liver.

(2) The medicament according to the above (1), wherein the steatohepatitis is developed by ATP8B 1 function decline in the intestinal tract.

(3) The medicament according to the above (1), wherein the steatohepatitis is provoked by progressive familial intrahepatic cholestasis type 1 (PFIC1), short bowel syndrome, inflammatory bowel disease, or obesity.

(4) The medicament according to the above (1), wherein the choline metabolite is betaine, dimethylglycine, or phosphatidylcholine.

(5) The medicament according to any one of the above (1) to (3), which is a preventive agent.

(6) The medicament according to any one of the above (1) to (3), which is a therapeutic agent.

(7) A food and beverage composition, comprising, as an active ingredient, choline or a choline metabolite, ethanolamine or an ethanolamine metabolite, a salt thereof, or a solvate thereof, wherein the composition is for use in prevention or improvement assistance of steatohepatitis.

(8) The composition according to the above (7), wherein the steatohepatitis is developed by ATP8B 1 function decline in the intestinal tract.

(9) The according to the above (7), wherein the steatohepatitis is provoked by progressive familial intrahepatic cholestasis type 1 (PFIC1), short bowel syndrome, inflammatory bowel disease, or obesity.

(10) The composition according to the above (7), wherein the choline metabolite is betaine, dimethylglycine, or phosphatidylcholine.

It is to be noted that the preposition "to" used in the present description indicates a numerical value range including the numerical values located left and right of the preposition.

Advantageous Effects of Invention

According to the present invention, preventive and therapeutic agents and a therapeutic method for diseases developed by ATP8B1 function decline in intestinal epithelial cells are provided. Furthermore, according to the present invention, preventive and therapeutic agents and a therapeutic method for steatohepatitis developed by phospholipid deficiency or decreased phospholipid synthesis in the liver are provided.

Brief Description of Drawings

[Figure 1] Figure 1 shows the results of lipidome analysis of the small intestinal tissues of Atp8b1 E-KO mice. The enrichment of lipid subtypes in the lipidome analysis results was visualized by Kolmogorov-Smirnov (KS) running sum statistic. The KS statistic was calculated for each lipid subtype and was plotted as running sum (upper view). The heatmap in the middle view and the barcode in the lower view show the changes in individual lipid contents and the positions of lipids classified into the indicated subtypes, respectively.

[Figure 2] Figure 2 shows a biosynthetic pathway of phospholipids in the liver. It is to be noted that Figure 2 is cited from Figure 2 (partially modified) of Biochim Biophys Acta Biomembr. 2017 Sep; 1859(9 Pt B): 1558-1572.

[Figure 3] Figure 3 shows studies regarding the influence of Atp8b1 deficiency in small intestinal epithelial cells on a living body. Figure 3Ais a view showing an outline of the metabolic pathway of phosphatidylcholines (PCs) in a living body and metabolites thereof. Figure 3B shows differences in the abundances of choline, betaine, dimethylglycine and phosphorylcholine in the livers of a small intestinal epithelial cell-specific Atp8b1 knockout (Atp8b1 E-KO) mouse group and a control mouse group (wild type), which are shown in forest plotting. Each point indicates a difference in the abundance between the Atp8b1 E-KO mouse group and the control group, and the bars indicate 95% confidence intervals.

[Figure 4] Figure 4 shows the results of producing an ATP8B 1 KO Caco-2 cell line. The knockout of ATP8B1 in Caco-2 cells was confirmed by Western blotting using an anti-ATP8B1 antibody. The Western blotting of ATP1A1 was also performed as a control. The term "parent" indicates the results of a Caco-2 wild line, and #116, #130, #133 and #171 indicates the results of the ATP8B1 KO Caco-2 cell line.

[Figure 5] Figure 5 shows the results obtained by examining the influence of lysophospholipids on the ATP8B1 KO Caco-2 cell line. Lysophosphatidylcholine (LysoPC), lysophosphatidylethanolamine (LysoPE), and edelfosine used as a control, were added to the ATP8B1 KO Caco-2 cells, and the degree of cytotoxicity was then examined.

[Figure 6] Figure 6 shows the results obtained by confirming that ATP8B1 functions as a flippase. The results obtained by measuring the amounts of NBD-labeled PC (phosphatidylcholine) (lower left view) and NBD-labeled LysoPC (lysophosphatidylcholine) (lower right view) incorporated into the cytoplasm of CHO-K1 cells by flow cytometry are shown. EV indicates the results obtained by transfecting an empty vector into cells, and ATP8B1+CDC50A indicates the result obtained by transfecting an ATP8B1 expression vector and a CDC50A expression vector into cells.

[Figure 7] Figure 7 shows studies (1) regarding the effect of feeding mother mice with a choline supplemented diet on hepatic lesions in pups. The results obtained by feeding Atp8b1 E-KO mother with a 1% choline supplemented diet, and measuring body weight, small intestine length and liver weight, at a time point when their pups reached 4 weeks of age, are shown. The term "Vehicle" indicates the results obtained when the pups were fed with a normal diet without choline supplementation, and the term "Choline" indicates the results obtained when the pups were fed with a 1% choline supplemented diet (the same applies in the following figures). The value indicates a mean value $\pm$ standard error. Regarding the Vehicle group, n = 11 for control (wild-type mice) and n = 14 for E-KO (Atp8b1 E-KO); and regarding the Choline group, n = 18 for control and n = 13 for E-KO (the same applies in the following figures). *: $p<0.05$, **: $p<0.01$, ***: $p<0.001$ (the same applies in the following figures).

[Figure 8] Figure 8 shows studies (2) regarding the effects of feeding mother mice with a choline supplemented diet on hepatic lesions in pups. The results obtained by measuring the liver injury markers (AST and ALT) in pups generated from Atp8b1 E-KO mother mice fed with a 1% choline supplemented diet are shown.

[Figure 9] Figure 9 shows studies (3) regarding the effects of feeding mother mice with a choline supplemented diet on hepatic lesions in pups. The hematoxylin and eosin staining images of the liver sections of pups generated from Atp8b1E-KO mother mice fed with a 1% choline supplemented diet are shown.

[Figure 10] Figure 10 shows studies (4) regarding the effects of feeding mother mice with a choline supplemented diet on hepatic lesions in pups. The abundance ratio (%) of tissue macrophages (F4/80 positive cells) and neutrophils (MPO positive cells) per field of view in the liver of pups generated from Atp8b1 E-KO mother mice fed with a 1% choline supplemented diet is shown. The F4/80 positive cells and the MPO positive cells were each visualized by a fluorescent tissue immunostaining method.

[Figure 11] Figure 11 shows studies regarding the influence of feeding mother mice with a choline supplemented diet on choline and metabolites thereof in the plasma of pups. The results obtained by measuring the concentrations of choline, phosphorylcholine, glycerophosphorylcholine, betaine, and dimethylglycine in the plasma of pups generated from Atp8b1 E-KO mother mice fed with a 1% choline supplemented diet are shown.

[Figure 12] Figure 12 shows studies (2) regarding the influence of feeding mother mice with a choline supplemented diet on choline and metabolites thereof in the liver of pups. The results obtained by measuring the concentrations of choline, phosphorylcholine, glycerophosphorylcholine, betaine and dimethylglycine in the liver of pups generated from Atp8b1 E-KO mother mice fed with a 1% choline supplemented diet are shown.

[Figure 13] Figure 13 shows studies (2) regarding the influence of feeding mother mice with a choline supplemented diet on choline and a metabolite thereof in the liver of pups. The results obtained by measuring the concentration of phosphatidylcholine (PC) in the liver of pups generated from Atp8b1 E-KO mother mice fed with a 1% choline supplemented diet are shown.

[Figure 14] Figure 14 shows studies regarding the influence of feeding mother mice with a choline supplemented diet on choline and metabolites thereof in the small intestinal epithelial cells of pups. The results obtained by measuring the concentrations of choline, phosphorylcholine, glycerophosphorylcholine and betaine in the small intestinal epithelial cells of pups generated from Atp8b1 E-KO mother mice fed with a 1% choline supplemented diet are shown.

[Figure 15] Figure 15 shows the results of the analysis using Tax-iIEC-KO mice (1). Figure 15(A) shows the results obtained by measuring the body weight and small intestine length of Tax-iIEC-KO mice at initiation of Tamoxifen administration (Day 0) and 14 days after initiation of the administration (Day 14); Figure 15(B) shows the results obtained by measuring the values of the liver injury markers (AST and ALT); and Figure 15(C) shows the pathological images of the liver. Tamoxifen (1 mg/day) was administered for 5 consecutive days starting at 8 weeks of age. Littermate Flox/Flox mice were used as controls.

[Figure 16] Figure 16 shows the results of the analysis using Tax-iIEC-KO mice (2). Figure 16(A) shows the pathological images of the small intestine of Tax-iIEC-KO mice at the start of Tamoxifen administration (Day 0) and 14 days after initiation of Tamoxifen administration (Day 14); and Figure 16(B) shows the results obtained by measuring villi length in the lower part of the small intestine. Tamoxifen (1 mg/day) was administered for 5 consecutive days starting at 8 weeks of age. Littermate Flox/Flox mice were used as controls.

[Figure 17] Figure 17 shows the results of the analysis using Tax-iIEC-KO mice (3). The results obtained by measuring the concentrations of choline, betaine and dimethylglycine in the liver of Tax-iIEC-KO mice 14 days after initiation of Tamoxifen administration (Day 14) are shown. Tamoxifen (1 mg/day) was administered for 5 consecutive days starting at 8 weeks of age. Littermate Flox/Flox mice were used as controls. ****: $p<0.0005$

[Figure 18] Figure 18 shows studies (1) regarding the effective amount of choline on hepatic lesions in Tax-iIEC-KO mice. Eight-week-old Tax-iIEC-KO mice or control mice (littermate Flox/Flox mice) were started to be fed with a control diet, a 0.3% choline supplemented, or a 1% choline supplemented diet. At the same time, 1 mg/day of Tamoxifen was administered to the mice for 5 consecutive days. Feeding of the mice with the control diet, the 0.3% choline supplemented or the 1% choline supplemented diet was continued, and at 10 weeks of age, the body weight and small intestine length were measured (Figure 18A), the values of the liver injury markers (AST and ALT) were measured (Figure 18B); and the pathological images of liver sections were observed (Figure 18C).

[Figure 19] Figure 19 shows studies (2) regarding the effective amount of choline on hepatic lesions in Tax-iIEC-KO mice. Eight-week-old Tax-iIEC-KO mice or control mice (littermate Flox/Flox mice) were started to be fed with a control diet, a 0.3% choline supplemented, or a 1% choline supplemented diet. At the same time, 1 mg/day of Tamoxifen was administered to the mice for 5 consecutive days. Feeding of the mice with the control diet, the 0.3% choline supplemented or the 1% choline supplemented diet was continued, and at 10 weeks of age, the concentrations of choline and betaine in the liver were measured.

[Figure 20] Figure 20 shows studies (3) regarding the effective amount of choline on hepatic lesions in Tax-iIEC-KO mice. Eight-week-old Tax-iIEC-KO mice or control mice (littermate Flox/Flox mice) were started to be orally administered with a normal saline, or with 50 mg/kg, 150 mg/kg or 500 mg/kg choline, three times a day. At the same time, 1 mg/day of Tamoxifen was administered to the mice for 5 consecutive days. Oral administration with the normal saline or the 50 mg/kg, 150 mg/kg or 500 mg/kg choline three times a day was continued, and at 10 weeks of age, the body weight and small intestine length were measured (Figure 20A), the values of the liver injury markers (AST and ALT) were measured (Figure 20B); and the concentrations of choline and betaine in the liver were measured (Figure 20C).

[Figure 21] Figure 21 shows the results of the analysis using clinical specimens (1). It shows the results obtained by measuring the abundance of choline (Choline), betaine (Betaine) and dimethylglycine (DMG) present in plasma samples from healthy subjects (Healthy), PFIC1 patients (PFIC1_Pre LTx), PFIC1 patients with orthotopic liver transplantation (PFIC1_Post LTx), other intrahepatic cholestasis patients (Other cholestasis Pre LTx), and other intrahepatic cholestasis patients who underwent orthotopic liver transplantation (Other cholestasis Post LTx).

[Figure 22] Figure 22 shows the results of the analysis using clinical specimens (2). It shows the results obtained by measuring the abundance of choline (Choline) and betaine (Betaine) in blood samples from healthy children (Healthy), PFIC1 patients (PFIC1), patients with inflammatory bowel disease (IBD), patients with short bowel syndrome (SBS), and obese children (Obese).

Description of Embodiments

Hereafter, the embodiments for carrying out the present invention will be described.

A first embodiment relates to a medicament for steatohepatitis, comprising, as an active ingredient, choline or a choline metabolite, ethanolamine or an ethanolamine metabolite, a salt of choline or a choline metabolite, a salt of ethanolamine or an ethanolamine metabolite, a solvate of choline or a choline metabolite, or a solvate of ethanolamine or an ethanolamine metabolite, wherein the medicament for steatohepatitis is characterized in that the steatohepatitis is developed by phospholipid deficiency or decreased phospholipid synthesis in the liver (hereinafter also referred to as "the medicament according to the present embodiment").

In the present embodiment, the "steatohepatitis developed by phospholipid deficiency or decreased phospholipid synthesis in the liver" is not particularly limited, and a typical disease thereof may be, for example, steatohepatitis associated with diseases developed by function decline of ATP8B1 (e.g., ATP8B 1 in intestinal epithelial cells) in the

intestinal tract, such as progressive familial intrahepatic cholestasis type 1 (PFIC1). Except for PFIC3, PFIC is a disease that is characterized in that it exhibits symptoms of intrahepatic cholestasis with a normal serum γ-GTP value. This disease is manifested as prolonged jaundice that begins soon after birth, and cholestasis-involved liver failure progresses. As a result, the disease leads to cirrhosis and liver failure before adulthood. In addition, the extremely intense pruritus associated with cholestasis, which interferes with daily life, significantly reduces the quality of life of patients and their guardians. Currently, no effective therapeutic methods have been established for this disease. Some subtypes of the disease are completely cured by liver transplantation, but liver transplantation is a therapeutic method that imposes extremely large burdens physically and economically on patients. Therefore, it has been strongly desired to develop a novel internal medicine treatment for the present disease.

Other examples of "steatohepatitis developed by phospholipid deficiency or decreased phospholipid synthesis in the liver" may include steatohepatitis associated with short bowel syndrome, steatohepatitis associated with inflammatory bowel disease, steatohepatitis associated with obesity, steatohepatitis developed in those receiving intravenous nutrition, and steatohepatitis developed in premature low-birth-weight infants. Furthermore, the "steatohepatitis developed by phospholipid deficiency or decreased phospholipid synthesis in the liver" in the present embodiment may be steatohepatitis developed in those who are after the surgical operation of, for example, esophageal atresia, small intestinal atresia, congenital diaphragmatic hernia, biliary atresia and biliary dilatation, and before the start of lactation, those with mitochondrial dysfunction related to phospholipid synthesis, and those who take drugs that act on a choline metabolic pathway, such as methotrexate.

From the analysis using Atp8b1 E-KO mice, the present inventors have found that hepatic lesions (steatohepatitis, etc.) in the present mice are caused by a deficiency of choline or a metabolite thereof or ethanolamine or a metabolite thereof in a body, which is due to a decrease in phospholipid biosynthesis associated with a deficiency of phospholipid raw materials such as choline and a metabolite thereof and ethanolamine and a metabolite thereof in the liver.

The medicament according to the present embodiment can be used as a preventive agent and/or a therapeutic agent for steatohepatitis developed by a decrease in the synthesis of phospholipids in the liver, and choline that is one active ingredient of the present medicament is a compound represented by the following formula (1):

[Formula 1]

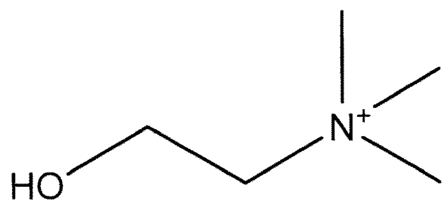

(1)

Moreover, examples of the metabolite of choline that is an active ingredient of the medicament according to the present embodiment may include, but are not particularly limited to, betaine, dimethylglycine (N,N-dimethylglycine), phosphorylcholine, and phosphatidylcholine. Among these, betaine (the following formula (2)), dimethylglycine (the following formula (3)), or phosphatidylcholine, etc. are preferable

[Formula 2]

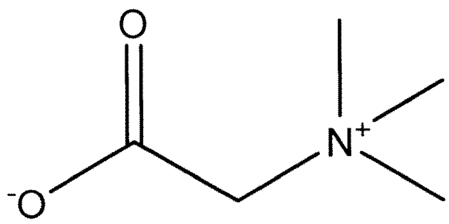

(2)

[Formula 3]

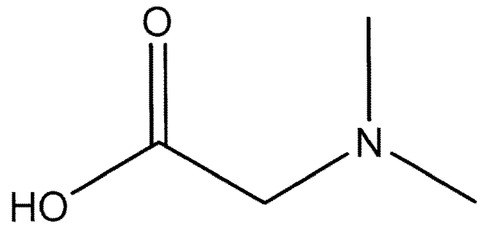

(3)

Ethanolamine that is one active ingredient of the medicament according to the present embodiment is a compound represented by the following formula (4):

[Formula 4]

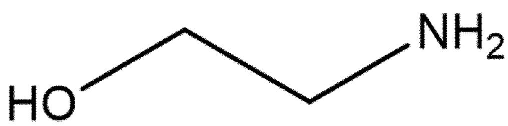

(4)

Moreover, examples of the metabolite of ethanolamine that is an active ingredient of the medicament according to the present embodiment may include, but are not particularly limited to, phosphoethanolamine and CDP-ethanolamine.

The medicament according to the present embodiment may be administered in a form in which a medicament for steatohepatitis serving as an active ingredient, namely, choline or a choline metabolite, ethanolamine or an ethanolamine metabolite, a salt of choline or a choline metabolite, a salt of ethanolamine or an ethanolamine metabolite, a solvate of choline or a choline metabolite, or a solvate of ethanolamine or an ethanolamine metabolite per se is administered. In general, however, the medicament according to the present embodiment may be administered in the form of a pharmaceutical composition in which one or two or more pharmaceutical additives are administered together with such an active ingredient (hereinafter also referred to as "the pharmaceutical composition according to the present embodiment"). Furthermore, the pharmaceutical composition according to the present embodiment may also comprise known other drugs.

The salt of the above-described choline or a metabolite thereof, or the salt of the above-described ethanolamine or a metabolite thereof, is preferably physiologically acceptable salt. When an acidic group is present, examples of such a salt include: alkaline metal and alkaline earth-metal salts such as lithium, sodium, potassium, magnesium or calcium; amine salts such as ammonia, methylamine, dimethylamine, trimethylamine, dicyclohexylamine, tris(hydroxymethyl)aminomethane, N,N-bis(hydroxyethyl)piperazine, 2-amino-2-methyl-1-propanol, ethanolamine, N-methylglucamine or L-glucamine; and salts formed with basic amino acids such as lysine, δ-hydroxylysine or arginine. When a basic group is present, examples of such a salt include: salts formed with mineral acids such as hydrochloric acid, hydrobromic acid, sulfuric acid, nitric acid or phosphoric acid; salts formed with organic acids such as methanesulfonic acid, benzenesulfonic acid, p-toluenesulfonic acid, acetic acid, propionic acid, tartaric acid, fumaric acid, maleic acid, malic acid, oxalic acid, succinic acid, citric acid, benzoic acid, mandelic acid, cinnamic acid, lactic acid, glycolic acid, glucuronic acid, ascorbic acid, nicotinic acid or salicylic acid; and salts formed with acidic amino acids such as aspartic acid or glutamic acid.

The medicament or pharmaceutical composition according to the present embodiment (hereinafter also referred to as "the medicament, etc. according to the present embodiment") may have either an oral or parenteral dosage form, and the dosage form thereof is not particularly limited. Examples of the dosage form may include a tablet, a capsule, a granule, a powder medicine, syrup, a suspension, a suppository, an ointment, a cream, a gel, a patch, an inhalant, and an injection. These pharmaceutical preparations are prepared according to ordinary methods. In the case of a liquid preparation, it may adopt a form in which the medicament is dissolved or suspended in water or another suitable medium before use. Moreover, in the case of a tablet or a granule, it may be coated according to a well-known method. In the case of an injection, it is prepared by dissolving the antibody of the present invention or a functional fragment thereof in water. The antibody of the present invention or a functional fragment thereof may also be dissolved in a normal saline or a dextrose solution, as necessary, and a buffer or a preservative may also be added to such a solution.

The types of pharmaceutical additives used in the production of the medicament, etc. according to the present embodiment, the ratio of the pharmaceutical additives to the active ingredient, or a method for producing the medicament

or the pharmaceutical composition may be appropriately determined by a person skilled in the art, depending on the form of the medicament or the pharmaceutical composition to be produced. As such pharmaceutical additives, inorganic or organic substances, or solid or liquid substances can be used. In general, such pharmaceutical additives can be added at a weight percentage of, for example, 0.1% by weight to 99.9% by weight, 1% by weight to 95.0% by weight, or 1% by weight to 90.0% by weight, based on the weight of the active ingredient. Specific examples of such pharmaceutical additives may include lactose, glucose, mannit, dextrin, cyclodextrin, starch, sucrose, magnesium aluminometasilicate, synthetic aluminum silicate, carboxymethylcellulose sodium, hydroxypropyl starch, carboxymethylcellulose calcium, ion-exchange resin, methyl cellulose, gelatin, gum Arabic, hydroxypropyl cellulose, hydroxypropylmethyl cellulose, polyvinylpyrrolidone, polyvinyl alcohol, light anhydrous silicic acid, magnesium stearate, talc, tragacanth, bentonite, VEEGUM, titanium oxide, sorbitan fatty acid ester, sodium lauryl sulfate, glycerin, fatty acid glycerin ester, purified lanolin, glycerinated gelatin, polysorbate, macrogol, vegetable oil, wax, liquid paraffin, white petrolatum, fluorocarbon, nonionic surfactant, propylene glycol, and water.

In order to produce a solid preparation used for oral administration, the active ingredient is mixed with an excipient ingredient such as, for example, lactose, starch, crystalline cellulose, calcium lactate or silicic acid anhydride to prepare a powder medicine. Otherwise, a binder such as saccharose, hydroxypropyl cellulose or polyvinylpyrrolidone, a disintegrator such as carboxymethyl cellulose or carboxymethylcellulose calcium, or other additives are further added to the above obtained mixture, as necessary, and the obtained mixture is then subjected to wet granulation or dry granulation, so as to prepare a granule. In order to produce a tablet, such a powder medicine or a granule may be subjected to a tablet-making operation, directly or with addition of a lubricant such as magnesium stearate or talc. The prepared granule or tablet may be coated with an enteric coating base such as hydroxypropylmethylcellulose phthalate or a methacrylic acid-methyl methacrylate polymer, so as to prepare an enteric coated drug. Otherwise, it may be coated with ethyl cellulose, carnauba wax or hydrogenated oil, so as to prepare a long-acting preparation. Moreover, in order to produce a capsule, a powder medicine or a granule is filled into a hard capsule, or the active ingredient is coated with gelatin, directly or after being dissolved in glycerin, polyethylene glycol, sesame oil, olive oil or the like, thereby producing a soft capsule.

In order to produce an injection, the active ingredient, together with a pH adjuster such as hydrochloric acid, sodium hydroxide, lactose, lactic acid, sodium, sodium monohydrogen phosphate or sodium dihydrogen phosphate, and an isotonizing agent such as sodium chloride or glucose, as necessary, is dissolved in a distilled water for injection, and the obtained solution is subjected to aseptic filtration and is then filled into an ampule. Otherwise, mannitol, dextrin, cyclodextrin, gelatin or the like may be further added to the above obtained solution, and the obtained mixture may be then subjected to vacuum freeze-drying, so as to prepare an injection which is dissolved before use. Furthermore, lecithin, polysorbate 80, polyoxyethylene hydrogenated castor oil or the like is added to the active ingredient, and it is emulsified in water, so as to prepare an emulsion for injection.

In order to produce a rectal administration agent, the active ingredient, together with a suppository base such as cacao butter, tri-, di- and mono-glyceride of fatty acid, or polyethylene glycol, is moisturized to be dissolved, and the resultant is then poured into a mold, followed by cooling. Otherwise, the active ingredient may be dissolved in polyethylene glycol or soybean oil or the like, and it may be then coated with a gelatin film.

The dose and administration frequency of the medicament, etc. according to the present embodiment are not particularly limited. These factors can be appropriately selected by a doctor's or pharmacist's judgment, depending on conditions such as the purpose of preventing and/or treating deterioration and/or progression of a disease to be treated, the type of the disease, and the body weight and age of a patient. In general, the dose of the present medicament is approximately 0.01 to 1,000 mg (the weight of the active ingredient) per adult per day via oral administration. The medicament can be applied once or divided over several administrations per day, or every several days. When the medicament is used as an injection, it is desirable that the medicament be continuously or intermittently administered at a dose of 0.001 to 100 mg (the weight of the active ingredient) per adult per day. More specifically, although it is not particularly limited, the dose of the present medicament is, for example, 10 mg (the weight of the active ingredient) or more, more preferably 50 mg (the weight of the active ingredient) or more, per kg of body weight, and the present medicament may be administered once or more per day, more preferably 3 times or more per day.

The medicament, etc. according to the present embodiment can be prepared as a sustained-release preparation such as an implantable tablet and a delivery system encapsulated into a microcapsule, using a carrier capable of preventing the sustained-release preparation from immediately being removed from the body. Examples of such a carrier that can be used herein include biodegradable and biocompatible polymers, such as ethylene vinyl acetate, polyanhydride, polyglycolic acid, collagen, polyorthoester, and polylactic acid. Such materials can be easily prepared by a person skilled in the art. In addition, a liposome suspension can also be used as a pharmaceutically acceptable carrier. Although it is not limited, but such a liposome can be prepared as a lipid composition comprising phosphatidyl choline, cholesterol and PEG-induced phosphatidylethanol (PEG-PE) by being passed through a filter with a suitable pore size, such that it has a size suitable for use, and it can be then purified by a reverse phase evaporation method.

The medicament, etc. according to the present embodiment can be provided in the form of a kit, together with

an instruction manual regarding an administration method, etc. The medicament or the pharmaceutical composition contained in the kit is supplied with a container made of a material that effectively keeps the activity of the active ingredient for a long period of time, does not adsorb the agent and the like on the inner surface of the container, and does not alter the constituents. For example, a sealed glass ampule may contain a buffer, etc. that is enclosed in the presence of neutral non-reactive gas such as nitrogen gas.

Moreover, the instruction manual of the present kit may be printed on a paper, etc., and/or may be stored on an electromagnetically readable medium, such as CD-ROM or DVD-ROM and it may be then provided in such a form.

A second embodiment relates to a food and beverage composition, comprising, as an active ingredient, choline or a choline metabolite, ethanolamine or an ethanolamine metabolite, a salt thereof, or a solvate thereof, wherein the composition is for use in prevention or improvement assistance of steatohepatitis (hereinafter also referred to as "the auxiliary composition according to the present embodiment").

The auxiliary composition according to the present embodiment is a composition to be auxiliarily ingested during the treatment period of steatohepatitis in order to enhance the therapeutic effects. For the term "steatohepatitis," please refer to the explanation of the first embodiment. The auxiliary composition according to the present embodiment may be in any form, and for example, when it is provided as a food and beverage composition, examples of the form of the present auxiliary composition may include: beverages such as soft drinks and nutritional drinks; confectioneries such as candy, gum, jelly, cream, and ice cream; dairy products such as dairy drink, fermented milk, yogurt drink, and butter; and other supplements. It can also be a seasoning for preparing meals during the treatment of steatohepatitis (therapeutic diet), according to the auxiliary composition to the present embodiment. Moreover, the auxiliary composition according to the present embodiment may also be a seasoning used for preparing meals (therapeutic diets) during the treatment of steatohepatiti s.

A third embodiment relates to a method for preventing or treating steatohepatitis developed by phospholipid deficiency or decreased phospholipid synthesis in the liver, wherein the method comprises administering the medicament, etc. according to the present embodiment to a patient.

Herein, the term "treat" means to stop or alleviate the progression and deterioration of the pathological conditions of the above-described disease in a patient who has already developed the disease, and it is a treatment for purpose of stopping or alleviating the progression and deterioration of the disease.

On the other hand, the term "prevent" means to stop the onset or development of the above-described disease in advance in a patient who is likely to be affected with the disease, and it is a treatment for the purpose of stopping the disease in advance.

When the present description is translated into English and the English description includes singular terms with the articles "a," "an," and "the," these terms include not only single items but also multiple items, unless otherwise clearly specified from the context that it is not the case.

Hereinafter, the present invention will be further described in the following examples. However, these examples are only illustrative examples of the embodiments of the present invention, and thus, are not intended to limit the scope of the present invention.

Examples

1. Methods

1-1. Materials and methods

The reagents used in the present Examples were of analytical grade. Caco-2 cells were obtained from Riken BioResource Research Center.

1-2. Animals and diets

All animal experiments were approved by and performed in accordance with the guidelines of the animal experiment committee of the University of Tokyo. To generate Atp8b1$^{flox/flox}$ mice, ICR and C57BL/6J mice were purchased from Charles River Laboratories International, Inc. (Yokohama, Japan). Mice were kept under pathogen-free conditions at a room temperature of 23.5°C ± 2.5°C and a relative humidity of 52.5% ±12.5% under a 14-hour light: 10-hour dark cycle. Mice had free access to standard chow diet (D10012G; Research diets, New Brunswick, NJ) and filtered water. In order to supplement choline to pups, mother mice were fed with a 1.0% choline supplemented diet (D20011404, Research Diets) until weaning of their pups.

1.3 Generation of Atp8b1 E-KO mice

Two genomic sequences (5'-GCAAGTGACTCAGACGTATG-3' (SEQ ID No: 1)) and (5'- CAGCTTGTAAGATCGCCTTG-3' (SEQ ID No: 2)) in the intron 2 and 3 of Atp8b1 were selected as guide RNA targets. Each sequence was inserted into pX330-mC plasmid, which carried both guide RNA and Cas9-mCdt1 (Mizuno-Iijima et al., Methods 191: 23-31, 2021). The flox donor plasmid DNA, pflox-Atp8b1, carried the genomic region from 2174 bp upstream to 1769 bp downstream of the exon 3 of Atp8b1. Two loxP sequences were inserted into a site between 908 bp upstream and 832 bp downstream of exon 3 of Atp8b1. For use in microinjection, these DNAs were isolated with FastGene Plasmid mini Kit (Nippon genetics, Tokyo, Japan) and were then filtrated by MILLEX-GV® 0.22 μm Filter unit (Merk Millipore, Darmstadt, Germany).

The pregnant mare serum gonadotropin (PMSG) (5 units) and the human chorionic gonadotropin (hCG) (5 units) were intraperitoneally injected into female C57BL/6J mice with a 48-hour interval, and the female C57BL/6J mice were then mated with male C57BL/6J mice. Thereafter, fertilized eggs were collected from oviducts in the mated female mice, and a mixture of pX330- pX330-mC (circular, 5 ng/μL, each) and pflox-Atp8b1 (circular, 10 ng/μL) was injected into fertilized eggs. Subsequently, survived fertilized eggs were transferred into oviducts in pseudopregnant female ICR mice, so as to obtain newborns.

To confirm whether or not a designed flox mutation was generated, the genomic DNA was extracted from the tail of the newborn pups using PI-200 (KURABO INDUSTREIS LTD, Osaka, Japan) according to manufacturer's protocols included therewith. Genomic PCR was performed using KOD-Fx (TOYOBO, Osaka, Japan). For checking correct loxP introduction and a large range of deletion mutations generated, the following primers were used:

Atp8b1 dflox long LeF:
5'-CGGGCTGCAGGAATTTGATGGCTATTTACATTCCCACCGTCTA-3' (SEQ ID No: 3); and
Atp8b1 dflox long RiR:
5'-ACGCATGTTTGAACGTCATGTCTAATTG-3' (SEQ ID No: 4).

Furthermore, the presence or absence of random integration of pX330-mC and pflox-Atp8b1 was checked by performing PCR using the following primers that detect an ampicillin resistance gene, and as a result, it was confirmed that no random integration was found in founder mice:

Amp detection-F: 5'-TTG CCG GGA AGC TAG AGT AA-3' (SEQ ID No: 5); and
Amp detection-R: 5'-TTT GCC TTC CTG TTT TTG CT-3' (SEQ ID No: 6).

Male mice harboring the designed floxed Atp8b1 allele with flox mutation were bred with female C57BL/6 mice, and the heterozygous floxed ($Atp8b1^{flox/+}$) mice in the obtained offsprings were intercrossed to produce homozygous floxed ($Atp8b1^{flox/flox}$) mice. The $Atp8b1^{flox/flox}$ mice were crossed with villin-Cre transgenic mice (Stock No: 021504; The Jackson Laboratory, Bar Harbor, ME), and $Atp8b1^{flox/+}$; villin-Cre mice in the obtained offsprings were mated with $Atp8b1^{flox/flox}$ mice to obtain intestinal epithelial cell (IEC)-specific Atp8b1 knockout (Atp8b1 IEC-KO; $Atp8b1^{flox/flox}$; villin-Cre) mice (Atp8b1 E-KO). Male Atp8b1 E-KO were mated with female Atp8b 1 $^{flox/flox}$ mice, and the resulting Atp8b1 E-KO mice and littermate $Atp8b1^{flox/flox}$ mice were used for animal experiments.

1-4. Preparation of Tamoxifen-induced Atp8b1 E-KO mice

Mice that are deficient in Atp8b1 specifically in intestinal epithelial cells by Tamoxifen administration (Atp8b1 Tax-iIEC-KO mice) were prepared as follows.

Atp8b1 $^{flox/flox}$ mice were mated with villin-Cre/ERT2 transgenic mice (Stock No. 020282; The Jackson Laboratory), and $Atp8b1^{flox/+}$;villin-Cre/ERT2 mice in the obtained offsprings were then mated with $Atp8b1^{flox/flox}$ mice to obtain Tamoxifen-induced, intestinal epithelial cell (IEC)-specific Atp8b1 knockout (Atp8b1Tax-iIEC-KO; Atp8b1 flox/flox;villin-Cre/ERT2) mice (Atp8b1 Tax-iIEC-KO mice). The resulting Atp8b1 Tax-iIEC-KO mice and littermate $Atp8b1^{flox/flox}$ mice were used for animal experiments.

1-5. Tissue sampling

Male 3 to 4-week-old intestinal epithelial cell-specific Atp8b1 knockout (Atp8b1 E-KO) mice and control mice (littermate Flox/Flox mice) underwent laparotomy under isoflurane anesthesia. Blood was collected from inferior vena cava and was collected into EDTA-coated tubes. The blood was centrifuged at 1,700 × g for 15 minutes, and plasma was separated. Thereafter, the plasma was flash-frozen in liquid nitrogen and was stored at -80°C. The liver was excised and was then flash-frozen in liquid nitrogen, and thereafter, the thus frozen liver was stored at -80°C, or was used for

histological and electron microscopic analyses. The small intestine was excised, was then rinsed with cold PBS containing 0.5m M taurocholic acid, and was then used for histological and electron microscopic analyses, or for preparation of epithelial cells. To isolate epithelial cells from small intestine, TTDR kit (Becton Dickinson, Becton Drive Franklin Lakes, NJ) was used. According to the manufacturer's instructions included with the kit, the small intestine was opened longitudinally, was sliced into small fragments with a size of roughly 2 mm. The fragments were incubated with 1 × TTDR diluted with DMEM (11995-065, gibco) at 37°C for 30 minutes, and the resulting fragments were then mixed with 2 mM EDTA/PBS containing 1% BSA. The thus obtained mixture was filtrated through a 70 μm cell strainer (VWR21008-952; Corning Inc, Corning, NY), and was then centrifugated at 250 × g at 4°C for 10 minutes. The obtained cell pellets were incubated with 1 × Pharm Lyse (Becton Dickinson, Becton Drive Franklin Lakes, NJ) at room temperature for 15 minutes for the lysis of red blood cells, and the resultant was then mixed with 2 mM EDTA/PBS containing 1% BSA. The thus obtained mixture was centrifuged at 250 × g at 4°C for 10 minutes. The prepared epithelial cells were flash frozen in liquid nitrogen, and were then stored at -80°C.

1-6. Human test specimens

The present study was approved by the institutional review boards at all participating facilities, and was then performed in accordance with the 1964 Declaration of Helsinki and its later amendments or comparable ethical standards (as revised in Edinburgh 2000). Informed consent was obtained from all patients or their parents (when the subjects were under 18 years of age) before enrollment in the study. In a case where it was difficult to obtain informed consent due to reasons such as the termination of the research subject's hospital visit, instead of omitting informed consent, it was ensured that the research subject had an opportunity to refuse participation in the study by posting information about the implementation of the study, including the purpose of the study, on the website of the participating institution.

Peripheral blood samples derived from patients with normal-GGT PFIC and control subjects with the same age (including healthy individuals, obese individuals, and those with pancreatitis, HBV, HCV, Alagille syndrome, citrullinemia, and biliary atresia) were collected in EDTA-coated blood sampling tubes (Becton Dickinson, Tokyo, Japan), and plasma was then prepared.

1-7. Biochemical experiment

ALT and AST were determined by automatic measurement with Dri-chem (FUJIFILM, Tokyo, Japan).

1-8. Histological analysis and immunohistochemistry

The liver was cut into small pieces, was then fixed with 10% neutral buffered formalin, and was then embedded in paraffin. The small intestine was rinsed with PBS, was fixed with 10% neutral buffered formalin, was then cut into 2 segments (corresponding to the proximal side and the distal side), and was then opened longitudinally. The thus cut small intestine was opened with a toothpick, was immobilized with a pathological pin, was then fixed with 10% neutral buffered formalin, and was then embedded in paraffin. Paraffin sections (3 μm each) were prepared, and were then deparaffinized and rehydrated. Thereafter, the resulting paraffin sections were stained with hemotoxylin and eosin, and were then mounted in Entellan new (Merck, Branchburg, NJ).

Upon performing immunohistochemical analysis, the rehydrated paraffin sections were subj ected to an antigen retrieval treatment with 1 × Tris-HCl (pH 10) (Sigma-Aldrich, St. Louis) in Decloaking Chamber NxGen (BIOCARE Medical, Concord, CA) for 20 minutes. Thereafter, nonspecific binding was blocked with 3% BSA/PBS at room temperature for 1 hour. The sections were incubated with primary antibodies at 4°C overnight, and were then incubated with Alexa Fluor-bound secondary antibodies at room temperature for 1 hour. The used primary and secondary antibodies were listed in Table 1. After mounting with ProLong Diamond Antifade mountant (Thermo Fisher, Waltham, WA), microscopic images were obtained by a Zeiss Axio Scan. Z1 (Carl Zeiss, Jena, Germany), and were processed on Zen 3.0 software (Carl Zeiss). To quantify immunostained area, the digital images were analyzed by ImageJ software (ver. 1.53c; National Institutes of Health, Bethesda, MD).

[Table 1]

| Antibodies | Manufacture | Identifier |
|---|---|---|
| Rat anti-F4/80 | Biolegend | 123101 |
| Mouse anti-iNOS | R&D Systems | MAB9502 |
| Alexa Fluor 488 donkey anti-mouse IgG | Thermo Fisher Scientific | A21202 |

(continued)

| Antibodies | Manufacture | Identifier |
|---|---|---|
| Alexa Fluor 488 donkey anti-rat IgG | Thermo Fisher Scientific | A21208 |
| | | |

1-9. Analysis of metabolites

The profiles of metabolites were acquired by hydrophilic interaction liquid chromatography/tandem mass spectrometry (HILIC/MS/MS), gas chromatography/tandem mass spectrometry (GC/MS/MS) analyses, and non-targeted lipidomic analysis.

1-9-1. HILIC/MS/MS analysis

Metabolites were extracted from plasma, liver, and intestinal epithelial cells with methanol. After centrifugation at 20,000 × g at 4°C for 5 minutes, the supernatant was mixed with 400 mM ammonium sulfate at a ratio of 19 : 1. The mixture was centrifuged at 20,000 × g at 4°C for 5 minutes. The supernatant was applied to the LC/MS/MS system, which consisted of a UHPLC Nexera liquid chromatography system (Shimadzu co., Kyoto, Japan) and a 5500QTRAP mass spectrometer (AB Sciex pte. ltd., Toronto, Canada). The analytes were passed through a ZIC-cHILIC column (2.1 × 100 mm, 3 μm, Merck Millipore, Darmstadt, Germany) at a temperature of 30°C with a gradient elution of the mobile phases, 10 mM ammonium formate aqueous solution and acetonitrile at a flow rate at 0.4 mL/min. The eluent was ionized using electrospray ionization and was scanned with an MRM mode (see Yuan et al., Nat. Protoc. 2012, 7 (5), 872-881. https://doi.org/10.103 8/nprot.2012.024). Each MRM peak was assigned to a target molecule in comparison with its authentic standard.

1-9-2. GC/MS/MS analysis

Metabolites were extracted from plasma, liver, and intestinal epithelial cells with methanol. After centrifugation at 20,000 × g at 4°C for 5 minutes, internal standards labelled with stable isotopes were added to the supernatant and dried under a stream of nitrogen gas. The dried sample was derivatized by oximation and trimethylsilylation. The derivatized metabolites were analyzed by an Agilent 7890A series gas chromatography system. Chromatographic separation was performed in a J&W Scientific DB-5MS-DG column (30 m × 0.25 mm i.d., df = 0.25 μm; Agilent Technologies Inc., Santa Clara, CA) by a temperature gradient with helium gas flow at a rate of 1 mL/min. The eluted metabolites were introduced into an Agilent 7010B triple-quadrupole mass spectrometer for electron impact ionization, and were then scanned at an MRM mode. The MRM data were processed by MassHunter (Agilent Technologies Inc., CA, USA).

1-9-3. Lipidome analysis

Lipidomic analysis was performed as previously described (Satomi, et al., J. Chromatogr. B Analyt. Technol. Biomed. Life. Sci. 2017, 1063, 93-100. https://doi.org/10.1016/j.jchromb.2017.08.020.). A sample was extracted with ethanol supplemented with 0.002% butylated hydroxy toluene, and was then centrifuged at 21,500 × g at 4°C for 5 minutes. The supernatant was injected into a CAPCELL PAK ADME column (2.1 × 100 mm, 2.7 μm; Shiseido, Kyoto, Japan) and was maintained at a temperature of 60°C, and the lipids were separated by gradient elution of aqueous mobile phase (Milli Q water containing 0.01% acetic acid, 1 mM $NH_3$, and 10 μM EDTA-2Na) and organic solvent mobile phase (0.001% acetic acid and 0.2 mM $NH_3$ in ethanol/isopropanol (1 : 1)], at a flow rate set to 0.7 mL/min. The eluents were analyzed using a Q Exactive HF-X Mass Spectrometer (Thermo Fisher Scientific, Inc., San Jose, CA). Their mass spectra were acquired in the data-dependent mode. Precursor- and product-derived ion spectra by higher energy collisional dissociation (HCD) were scanned with an orbitrap analyzer at a resolution of 120,000 and 7,500 full width at half maximum at 200 m/z. The raw LC/MS data were processed by Expressionist Refiner MS software (ver. 8.2, Genedata AG, Basel, Switzerland). Each MS peak was compared with the in-house lipid database including information of retention time, exact mass, the preferred adduct ion species, and its structure was estimated.

1-9-4. Measurement of concentrations of choline metabolites

The concentrations of choline, betaine, dimethylglycine (N,N-dimethylglycine) and glycerophosphorylcholine (sn-glycero-3-phosphocholine) in plasma were determined by liquid chromatography/tandem mass spectrometry

(LC/MS/MS) assay. The sample was mixed with 98-fold volume of ethanol and 1-fold volume of the internal standard (IS) solution (10 μmol/L choline in water, 10 μmol/L glycerophosphorylcholine in water, and 1 μmol/L betaine in water). After vortex mixing, the sample was centrifuged at 21,500 × g at 4°C for 5 minutes. The obtained supernatant was injected into a UHPLC Nexera liquid chromatography system (Shimadzu co., Kyoto, Japan) equipped with an Ascentis Express HILIC column (2.1 × 50 mm, 2.7 μm, Merck KGaA, Darmstadt, Germany), and was then maintained at 4°C. The analyte was separated from the internal standard by gradient elution of mobile phase A (10 mmol/L ammonium acetate in water) and of mobile phase B (acetonitrile/water mixture) (99:1, v/v)). The gradient elution was carried out under the following conditions: 0-2 min., 82.5% B; 2-4 min., 82.5% to 5% B and 4-7 min. 5% B with a flow rate of 0.6 mL/min. The eluate was directly subjected to electrospray ionization under a positive ion mode using a 5500QTRAP mass spectrometer (Sciex pte. ltd., Toronto, Canada). All target molecules were scanned by selected reaction monitoring (SRM) mode. The conditions for performing SRM are shown in Table 2.

[Table 2]

| Compound Name | Q1 | Q3 | Dwell time | DP | EP | CE | CXP |
|---|---|---|---|---|---|---|---|
| Choline | 104 | 60 | 100 | 90 | 10 | 21 | 10 |
| Betaine | 118 | 59 | 100 | 90 | 10 | 41 | 10 |
| N,N-dimethylglycine | 104 | 58 | 100 | 70 | 10 | 21 | 10 |
| sn-glycero-3-phosphocholine | 258.1 | 104 | 100 | 60 | 10 | 51 | 10 |
| Choline-IS | 113 | 69 | 100 | 90 | 10 | 21 | 10 |
| Betaine-IS | 124 | 62 | 100 | 90 | 10 | 51 | 10 |
| Phosphocholine-IS | 193 | 95 | 100 | 45 | 10 | 25 | 10 |
| Abbreviations) DP: declustering potential (volts), EP: entrance potential (volts), CE: collision energy (volts), CXP: collision cell exit potential (volts), Dwell time (msec.). | | | | | | | |

Regarding the calibration curve, working solutions for calibration curve were prepared as follows. A stock solution of the working solution for calibration curve was diluted to concentration of 0.5, 1, 2.5, 5, 10, 25, 50, 100 and 250 μmol/L for choline, betaine and sn-glycero-3-phosphocholine, and 0.05, 0.1, 0.25, 0.5, 1, 2.5, 5, 10 and 25 μmol/L for N,N-dimethylglycine in ethanol. A working solution (10 μL) for calibration curve was mixed with 10 μL of an internal standard solution and 10 μL of water, and 970 μL of ethanol was then added to the mixed solution. Thereafter, the obtained mixture was subjected to the aforementioned electrospray ionization analysis. The linear calibration curve was drawn with a weighting of 1/x, and it was confirmed that the accuracy was within a range of ± 20%. The SRM data were processed by MultiQuant 3.0.2 (Sciex, pte. ltd., Toronto, Canada).

1-10. Enrichment of lipid subtypes in lipidome analysis results of small intestinal epithelium of Atp8b1 E-KO mice

The enrichment of lipid subtypes in the results of lipidome analysis was visualized by the Kolmogorov-Smirnov (KS) running sum statistic. Regarding individual tissues, the change in lipid amounts induced by Atp8b1-knock-in was scored as a difference from a control group as follows.

[Equation 1]

$$score_{l,i} = \frac{x_{l,i} - \mu_{control,l}}{\sigma_{control,l}}$$

wherein $x_{l,i}$ represents the amount of lipid l in the $i^{th}$ sample of the Atp8b1-knock-in group, and $\mu_{control,l}$ and $\delta_{control,l}$ represent the mean value and standard deviation of the amount of lipid l in the control group.

1-11. Preparation of ATP8B1 knockout (ATP8B1 KO) Caco-2 cells

Caco-2 cells were cultured in DMEM (Dulbecco's modified Eagle's medium) (11995-065, Thermo Fisher Scientific) supplemented with 10% fetal bovine serum (10437, Thermo Fisher Scientific), 1% nonessential amino acids

(11140-050, Thermo Fisher Scientific), 1% penicillin and streptomycin (15140-122, Thermo Fisher Scientific). The cells were cultured under conditions of 37°C, 5% $CO_2$ and a humidity of 95%.

To delete the ATP8B1 gene in Caco-2 cells, crRNA (5'-TAGGGAACCACTTCGTCATT-3' (SEQ ID No: 7), Integrated DNA Technologies; IDT) targeting the ATP8B1 gene was prepared. Integrated DNA and tracrRNA (1072533, IDT) were mixed with each other to a final concentration of 50 μM each. The mixture was heat-treated at 95°C for 5 minutes, and then, the temperature was decreased to room temperature, so as to prepare gRNA. According to the manufacturer's instructions, the prepared gRNA was incubated with Cas9 enzyme (1081059, IDT) at room temperature for 20 minutes, and the reaction mixture was then mixed with Electroporation Enhancer (IDT, 1075916). The obtained mixture was added to Caco-2 cells in SE buffer (PBC1-02250, Lonza). The thus obtained mixture was introduced into the cells by electroporation using a Lonza 4D Nucleofector under the DG-113 program conditions.

After completion of the electroporation, the cells were recovered, and genomic DNA was then extracted using DNAdvance Kit (Beckman Coulter). The extracted genomic DNA was used to amplify the target region by PCR. The primers used in PCR are as follows:

Forward: 5'-GCAGCATGCAGGCAGTATTCAACC-3' (SEQ ID No: 8); and
Reverse: 5'-CGAGTTCACACCACTGGACTCCAG-3' (SEQ ID No: 9).

The PCR products were reannealed in NE buffer ((B7002S, New England Biolabs; NEB). The hybridized PCR products were treated with T7 endonuclease (M0302S, NEB) at 37°C for 1 hour, and were then analyzed using MultiNA (Shimazu). Cells that deleted the gene were allowed to grow, and were subjected to Western blotting in order to confirm that the ATP8B1 protein was deleted.

1-12. Cytotoxicity test

5 mM Edelfosine dissolved in ethanol (CAY-60912-10, Cayman), 10 mg/mL lysophosphatidylcholine (LysoPC) dissolved in water/ethanol (1 : 1, v/v) (123-03781, FUJIFII,M Wako Pure Chemical), and 10 mg/mL lysophosphatidylethanolamine (LysoPE) (L4754, Sigma-Aldrich) dissolved in ethanol were prepared as stock solutions. Caco-2 cells and ATP8B1 KO Caco-2 cells were seeded on 96-well plates at a cell density of 50,000 cells/well, and were then cultured, while replacing the medium with a fresh medium every 2 days. After 7 days of the culture, the cells were cultured for 24 hours in a new medium supplemented with various concentrations of edelfosine, lysophosphatidylcholine and lysophosphatidylethanolamine. Cytotoxicity was measured using, as an indicator, the amount of lactate dehydrogenase (LDH) released from the injured cells into the medium. The amount of LDH in the medium was measured using the Cytotoxicity LDH Assay Kit-WST (CK12, Dojindo) according to the instructions included therewith.

1-13. Flippase (ATP8B1) assay

The experiment regarding incorporation of NBD-phospholipids into cells was carried out according to the previous reports (Suzuki et al., Nature 468,834-838 2010; Takatsu et al., J. Biol. Chem., 289:33543-33556 2014).

To allow CDC50A alone, or ATP8B1 and CDC50A, to express in CHO-K1 cells, the CHO-K1 cells (CCL-61, ATCC, Manassas, VA) were transfected with the plasmids (pShuttle-EV, or pShuttle-ATP8B1-FLAG and pShuttle-HA-CDC50A), using PEI MAX (Polysciences, Warrington, PA). Forty-eight hours after the transfection, the cells were detached from dishes and were collected in PBS containing 5 mM EDTA, and thereafter, the cells were recovered in a precipitate by centrifugation. The cells ($2 \times 10^6$ cells/sample) were washed and were then equilibrated at 15°C for 15 minutes in 500 μL of HBSS (14025092, Thermo Fisher Scientific). An equal volume of 1 μg/mL 18:1-06:0 NBD-PC (810132, Avanti Polar Lipids) or 10 μg/mL 12:0 LysoNBD-PC (810128P; Avanti Polar Lipids) was added to the cell suspension, and the obtained mixture was then incubated at 15°C. Thereafter, at each time point, 300 μL of a cell suspension was collected, and was mixed with 300 μl of HBSS (ice cold) containing 5 % fatty acid-free BSA (A6003; Sigma-Aldrich) to extract NBD-lipids incorporated into the exoplasmic leaflet of the plasma membrane as well as unincorporated NBD-Lipids.

Next, 10,000 cells were analyzed with a BD FACSCelesta (BD Bioscience, San Jose, CA) to measure the fluorescence of NBD-lipids incorporated into the cytoplasmic leaflet of the plasma membrane. The mean value of the fluorescence intensities per cell was calculated. Propidium iodide (P378; DOJINDO, Kumamoto, Japan) positive cells were excluded from the analysis.

1-14. Statistical processing

Graphs include means ± standard error of the mean (SEM), unless otherwise indicated. Differences between two variables and multiple variables were assessed at the 95% confidence level, using Student t tests and analysis of

variance (ANOVA) with post hoc Tukey tests, respectively. Survival rate of each genotype was determined by Kaplan-Meier method. The data were analyzed using GraphPad Prism 6.07 (GraphPad Software, La Jolla, CA).

2. Results

2-1 Functional Analysis of Atp8b1

2-1-1. Analysis using Atp8b 1 knockout mice

In intestinal epithelial cell-specific Atp8b1 knockout (Atp8b1 E-KO) mice, the findings consistent with cholestasis-related liver injury, such as an increase in liver injury markers and a reduction in secondary bile acids, were observed, and in liver histopathological analysis, mild cholestasis was confirmed. In addition, the Atp8b1 E-KO mice exhibited morphological abnormality of the small intestine. These results suggest that the Atp8b1 E-KO mice can be utilized as PFIC1 mouse models.

Thus, to investigate the constitution of lipids in the small intestinal epithelial cells of the Atp8b1 E-KO mice, the small intestinal epithelial cells were detached and were subjected to lipidome analysis. As a result, it was suggested that lysophospholipids such as lysophosphatidylcholine (LPC) and lysophosphatidylethanolamine (LPE) were accumulated in the small intestinal epithelial cells of the Atp8b1 E-KO mice (Figure 1).

Deletion of Atp8b1 in the intestinal tract results in impaired uptake of LPC and LPE in intestinal epithelial cells (i.e., LPC and LPE are accumulated in the outer membrane of the cells and are not incorporated into the inner membrane). LPC and LPE are converted to phosphatidylcholine (PC) and phosphatidylethanolamine (PE) in the intestinal epithelial cells. Thereafter, PC and PE are transported to the liver via the blood in the portal vein. Since PC and PE are the major phospholipids in the liver (Figure 2), it is considered that deletion of Atp8b1 results in a decreased supply of phospholipids to the liver and the development of steatohepatitis.

Subsequently, metabolome analysis was performed on the liver of Atp8b1 E-KO mice as a target. Figure 3B shows a difference in the abundances of individual metabolites in the liver of the Atp8b1 E-KO mouse group and the control mice (wild type) group, as a forest plot. Figure 3B suggests that the concentrations of LPC metabolites, namely, choline, betaine, dimethylglycine and phosphorylcholine are decreased. The major source of choline in the body is not de novo, but the metabolized form of phosphatidylcholine (PC) ingested by the diet. Therefore, the results of the aforementioned lipidome analysis and the results shown in Figure 3 suggest that the incorporation and metabolism of LPC in the small intestine of the Atp8b1 E-KO mice are decreased, and as a result, the amounts of choline and its metabolite group in the body are decreased. Since choline is utilized for the biosynthesis of PC in the liver, the present inventors have conceived that when some functional disorder (functional decline, etc.) occurs on ATP8B1 in intestinal epithelial cells, phospholipid raw materials (choline and ethanolamine) are depleted in the liver, and as a result, a decrease in phospholipid synthesis causes the onset of steatohepatitis.

2-1-2. Analysis using ATP8B1 KO Caco-2 cell line

Caco-2 cells are a cell line derived from human colon cancer and are used as cell models to reproduce intestinal tract functions in the human small intestine. In order to confirm that ATP8B 1 was knocked out, crude membrane fractions were prepared from the knockout cell line and were then subjected to Western blotting using an anti-ATP8B1 antibody. As a result, ATP8B 1 knockout was confirmed in clone #116, clone #130, clone #133 and clone #171 cell lines (Figure 4).

Next, the influence of lysophospholipids on ATP8B1 KO Caco-2 cells was examined. It was confirmed that, in the wild-type Caco-2 cell line, the addition of LPC or LPE to the culture medium did not cause significant cytotoxicity, but that in the ATP8B1 KO Caco-2 cell line, the addition of LPC or LPE increased cytotoxicity in a dose-dependent manner (Figure 5).

On the other hand, edelfosine, which was used as a control, is an analog of LPC but is not a substrate of ATP8B1. Accordingly, it is considered that edelfosine is retained in the extracellular membrane, disrupts lipid rafts and inhibits the function of elements essential for cell survival (e.g., proton pump, etc.), and exhibits cytotoxicity. As shown in Figure 5, edelfosine exhibited cytotoxicity against both wild-type cell lines and knockout cell lines.

From these results, it is considered that, in the ATP8B1 KO cell line, LPC and LPE are retained in the extracellular membrane because ATP8B 1 does not function as a flippase, and thus, disrupt lipid rafts and exhibit cytotoxicity.

2-1-3. Analysis of function of ATP8B1 as a flippase

In 2-1-2 above, it was shown that LPC and LPE exhibit cytotoxicity against the ATP8B 1 KO cell line. To corroborate this result, an experiment was carried out to confirm that ATP8B 1 functions as a flippase, i.e., that ATP8B1 functions as a flippase to incorporate phospholipids into the cell.

CDC50A alone, or ATP8B 1 and CDC50A were allowed to express in CHO-K1 cells. ATPB 1 is expressed in a functional state in the plasma membrane by forming heterodimers with CDC50A. When NBD-labeled phosphatidylcholine (NBD-PC) or NBD-labeled lysophosphatidylcholine (NBD-LysoPC) was added to these cells, in CHO-K1 cells expressing functional ATP8B 1 (Figure 6, ATP8B1+CDC50A), both NBD-PC and NBD-LysoPC were incorporated into the cells. In contrast, it was found that, in CHO-K1 cells without ATP8B1 expression (Figure 6, EV), both NBD-PC and NBD-LysoPC were incorporated into the cells in reduced amounts.

From the aforementioned results and the results of 2-1-2 above, it was suggested that ATP8B1 in intestinal epithelial cells is responsible for the intracellular incorporation of phospholipids such as PC and LPC (LysoPC) existing in the intestinal lumen into the cytoplasm.

2-2. Studies regarding defective phospholipid synthesis in liver, using Atp8b1 E-KO mice

2-2-1. Effects of feeding mother mice with choline supplemented diet on hepatic lesions of pups

Since a choline-deficient diet induces a decrease in phospholipid synthesis in the liver, it is a common method for producing fatty liver mouse models.
Accordingly, it was assumed that the hepatic lesions in Atp8b1 E-KO mice were related to steatohepatitis associated with phospholipid deficiency. Thus, mother mice were fed with a 1% choline supplemented diet, and thereafter, their pups were dissected when they reached 4 weeks of age. No changes were observed in body weight and small intestine length by feeding with the 1% choline supplemented diet, but an increase in liver weight observed in Atp8b 1 E-KO pups was resolved by feeding the 1% choline supplemented diet to the mother mice (Figure 7). In addition, in pups generated from mother mice fed with a choline supplemented diet (hereafter referred to as "choline supplemented diet-fed pups"), a decrease in liver injury markers (Figure 8, AST and ALT ) was found and normalization of liver pathological images was observed (Figure 9). Furthermore, the amounts of tissue macrophages (F4/80 positive cells) and neutrophils (MPO positive cells) were reduced in the livers of the choline supplemented diet-fed pups (Figure 10), and it was suggested that liver inflammation was improved by ingestion of choline.

From these results, it became clear that hepatic lesions in Atp8b1 E-KO mice are caused by a deficiency of choline and a metabolite group thereof in the liver.

2-2-2. Effects of feeding mother mice with choline supplemented diet on choline and metabolites thereof in vivo in pups

Mother mice were fed with a 1% choline supplemented diet, and thereafter, their pups were dissected when they reached 4 weeks of age. Changes in the concentrations of choline and metabolites thereof in plasma, liver, and small intestinal epithelial cells. and metabolites of choline in plasma, liver, and small intestinal epithelial cells were examined.

2-2-3. Concentrations of choline and choline metabolites in plasma

The concentrations of choline and metabolites thereof in the plasma of choline supplemented diet-fed pups were measured by LC/MS/MS. As a result, regarding choline, phosphorylcholine, and glycerophosphorylcholine (GPC), the concentrations thereof in the plasma of the choline supplemented diet-fed pups were not changed by feeding their mother mice with a 1% choline supplemented diet, as in the case of feeding the mother mice with a normal diet (a 0.1% choline-containing diet, Vehicle) (Figure 11).

On the other hand, regarding betaine and dimethylglycine, the concentrations of betaine and dimethylglycine in the plasma of the choline supplemented diet-fed pups were recovered by feeding mother mice with a 1% choline supplemented diet to the same levels as those in control mice (Figure 11).

From these results, it was suggested that feeding mother mice with a 1% choline supplemented diet increases the amounts of betaine and dimethylglycine in Atp8b1 E-KO pups and thereby suppresses the onset of hepatic lesions in the Atp8b1 E-KO pups.

2-2-4. Concentrations of choline and choline metabolites in liver

The concentrations of choline and metabolites thereof in the liver of choline supplemented diet-fed pups were measured by LC/MS/MS. As a result, regarding choline, phosphorylcholine, and glycerophosphorylcholine (GPC), the concentrations thereof in the liver of the choline supplemented diet-fed pups were not changed by feeding their mother mice with a 1% choline supplemented diet, as in the case of feeding the mother mice with a normal diet (a 0.1% choline-containing diet, Vehicle) (Figure 12).

On the other hand, regarding betaine, dimethylglycine, and phosphatidylcholine (PC), the concentrations of

betaine, dimethylglycine, and phosphatidylcholine (PC) in the liver of the choline supplemented diet-fed pups were recovered by feeding mother mice with a 1% choline supplemented diet to the same levels as those in control mice (Figure 12 and Figure 13).

From these results, it was suggested that feeding mother mice with a 1% choline supplemented diet increases the amounts of betaine, dimethylglycine, and phosphatidylcholine in Atp8b1 E-KO pups and thereby suppresses the onset of hepatic lesions in the Atp8b1 E-KO pups.

2-2-5. Concentrations of choline and choline metabolites in small intestinal epithelial cells

The concentrations of choline and metabolites thereof in the small intestinal epithelial cells of choline supplemented diet-fed pups were measured by LC/MS/MS. As a result, regarding choline, phosphorylcholine, and glycerophosphorylcholine (GPC), the concentrations thereof in the small intestinal epithelial cells of the choline supplemented diet-fed pups were not changed by feeding their mother mice with a 1% choline supplemented diet, as in the case of feeding the mother mice with a normal diet (a 0.1% choline-containing diet, Vehicle) (Figure 14). Likewise, the concentration of betaine in the small intestinal epithelial cells was not changed (Figure 14). Besides, regarding dimethylglycine, since the abundance thereof is the lower limit of quantification, dimethylglycine could not be detected.

2-3. Analysis of phospholipid synthesis in liver of Tax-iIEC-KO mice

Next, in order to directly analyze the effects of a choline supplemented diet on Atp8b1-deficient mice, studies were conducted using Tax-iIEC-KO mice.

2-3-1. Characterization of Tax-iIEC-KO mice

Transgenic mice expressing Cre specifically in intestinal epithelial cells by administration with Tamoxifen were mated with flox/flox mice to generate mice that delete Atp8b1 specifically in intestinal epithelial cells by administration with (Tax-iIEC-KO mice ). Eight-week-old Tax-iIEC-KO mice were administered with 1 mg/day Tamoxifen for 5 consecutive days and were then dissected at 10 weeks of age. In the Tax-iIEC-KO mice, in addition to weight reduction (upper view of Figure 15A) and small intestinal elongation (lower view of Figure 15A), an increase in liver injury markers was observed (Figure 15B). Moreover, in liver pathological analysis, the onset of steatohepatitis was confirmed (Figure 15C, Day 14 of Tax-iIEC-KO mice), and in small intestinal pathological analysis, the shortening of villi in the lower part of the small intestine was observed on Day 14 (Figure 16B, "Distal," Day 14). The observed shortening of villi in the lower part of the small intestine of mice lacking Atp8b1 suggests that ATP8B1 mainly functions in the lower part of the small intestine.

Subsequently, the amounts of choline and metabolites thereof in the liver were examined, and as a result, it was found that betaine and dimethylglycine were decreased (Figure 17), indicating that Tax-iIEC-KO mice exhibit a phenotype similar to that of IEC-KO mice.

2-3-2. Studies regarding effective amount of choline for steatohepatitis in Tax-iIEC-KO mice

In order to examine the concentration of choline required for suppression of steatohepatitis exhibited by Tax-iIEC-KO mice, the effect of a 0.3% choline supplemented diet on the suppression of steatohepatitis was evaluated. Eight-week-old Tax-iIEC-KO mice and control mice were started to be fed with a control diet, a 0.3% choline supplemented diet, or a 1% choline supplemented diet, and at the same time, the Tax-iIEC-KO mice and the control mice were administered with 1 mg/day of Tamoxifen for 5 consecutive days. Thereafter, the mice were dissected at 10 weeks of age. As a result, it was found that body weight and small intestine length were not changed with the choline supplemented diet (Figure 18A), but that in the 0.3% choline supplemented diet-fed group, an increase in liver injury markers was suppressed, as with the 1% choline supplemented diet-fed group (Figure 18B), and the onset of steatohepatitis was suppressed (Figure 18C). Moreover, the concentrations of choline and betaine in the liver were recovered in the 0.3% choline supplemented diet-fed group as well as in the 1% choline supplemented diet-fed group, to the same level as that in control mice (Figure 19).

Next, for the purpose of determining the dosage and administration of choline used for the treatment of patients with ATP8B1 function decline including PFIC 1, an effective dose evaluation test was carried out by oral administration of choline to Tax-iIEC-KO mice. Eight-week-old Tax-iIEC-KO mice and control mice were started to be orally administered with a normal saline, or with 50 mg/kg, 150 mg/kg or 500 mg/kg chloride choline, three times a day, and at the same time, the Tax-iIEC-KO mice and the control mice were administered with 1 mg/day of Tamoxifen for 5 consecutive days. Oral administration with a normal saline, or with 50 mg/kg, 150 mg/kg or 500 mg/kg chloride choline, three times a day, was continued, and thereafter, the mice were dissected at 10 weeks of age. The doses of 150 mg/kg and 500 mg/kg were determined by estimating the amount of choline consumed by the 0.3% choline supplemented diet group and the

1% choline supplemented diet group based on the daily food intake of mice. As a result, suppression of the body weight loss of the Tax-iIEC-KO mice (Figure 20A) and normalization of liver injury markers (Figure 20B) could be confirmed at the doses of 50 mg/kg, 150 mg/kg, and 500 mg/kg. Moreover, the concentrations of choline and betaine in the liver were recovered up to the same level as those in the control mice at all doses (Figure 20C).

2-4. Analysis using clinical specimens

The analysis using Atp8b1 E-KO mice suggested that hepatic lesions in the present mice are caused by a deficiency of choline and metabolites thereof (in particular, betaine and dimethylglycine) in the body. Thus, whether the present hypothesis of pathogenesis can be extrapolated to children with PFIC1 (caused by ATP8B1 deficiency) was examined using plasma samples from pediatric patients with liver diseases, including pediatric PFIC1 patients, as typical examples.

As a result, it became clear that the concentrations of choline, betaine, and dimethylglycine were significantly decreased in PFIC1 pediatric patients (PFIC1), compared with pediatric patients with other liver diseases (Figure 21; 2 and 3). Considering the findings found in the Atp8b1 E-KO mice, hepatic lesions in PFIC1 are likely to be treated by administration with choline, betaine, and dimethylglycine.

Furthermore, the concentrations of choline and betaine in the sera of 3 patients with inflammatory bowel disease and 3 patients with short bowel syndrome were determined by LC/MS/MS. In addition, since obese children also have fatty liver in some cases, the same analysis was performed on the sera of 10 obese children. As a result, it became clear that, in the inflammatory bowel disease group and the short bowel syndrome group, the blood choline concentration was decreased to the same level as that in PFIC1 (Figure 22). In the obese children as well, it was found that the blood choline concentration was also decreased to about half of that in healthy children (Figure 22).

Regarding betaine as well, the blood betaine concentration was decreased in inflammatory bowel disease and short bowel syndrome to the same level as that in PFIC1, and a trend of decrease was also observed in the obese children, compared with healthy children (Figure 22).

From these results, the effectiveness and possibility of choline and betaine in suppressing the onset and progression of fatty liver in children with these diseases was suggested.

Industrial Applicability

The present invention provides a preventive agent, a therapeutic agent, and a therapeutic method for steatohepatitis developed by phospholipid deficiency or decreased phospholipid synthesis in the liver (for example, steatohepatitis developed by ATP8B 1 function decline in intestinal epithelial cells). It is expected that the present invention will be utilized in the medical field.

## Claims

1. A medicament for steatohepatitis, comprising, as an active ingredient, choline or a choline metabolite, ethanolamine or an ethanolamine metabolite, a salt thereof, or a solvate thereof, wherein the medicament for steatohepatitis is **characterized in that** steatohepatitis is developed by phospholipid deficiency or decreased phospholipid synthesis in the liver.

2. The medicament according to claim 1, wherein the steatohepatitis is developed by ATP8B 1 function decline in the intestinal tract.

3. The medicament according to claim 1, wherein the steatohepatitis is provoked by progressive familial intrahepatic cholestasis type 1 (PFIC1), short bowel syndrome, inflammatory bowel disease, or obesity.

4. The medicament according to claim 1, wherein the choline metabolite is betaine, dimethylglycine, or phosphatidylcholine.

5. The medicament according to any one of claims 1 to 3, which is a preventive agent.

6. The medicament according to any one of claims 1 to 3, which is a therapeutic agent.

7. A food and beverage composition, comprising, as an active ingredient, choline or a choline metabolite, ethanolamine or an ethanolamine metabolite, a salt thereof, or a solvate thereof, wherein the composition is for use in prevention

or improvement assistance of steatohepatitis.

**8.** The composition according to claim 7, wherein the steatohepatitis is developed by ATP8B 1 function decline in the intestinal tract.

**9.** The composition according to claim 7, wherein the steatohepatitis is provoked by progressive familial intrahepatic cholestasis type 1 (PFIC1), short bowel syndrome, inflammatory bowel disease, or obesity.

**10.** The composition according to claim 7, wherein the choline metabolite is betaine, dimethylglycine, or phosphatidylcholine.

[Figure 1]

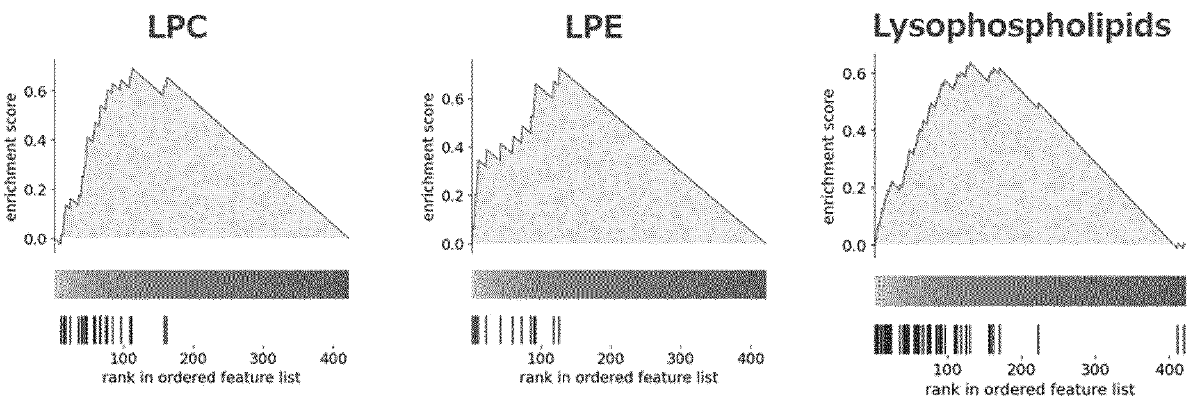


[Figure 2]

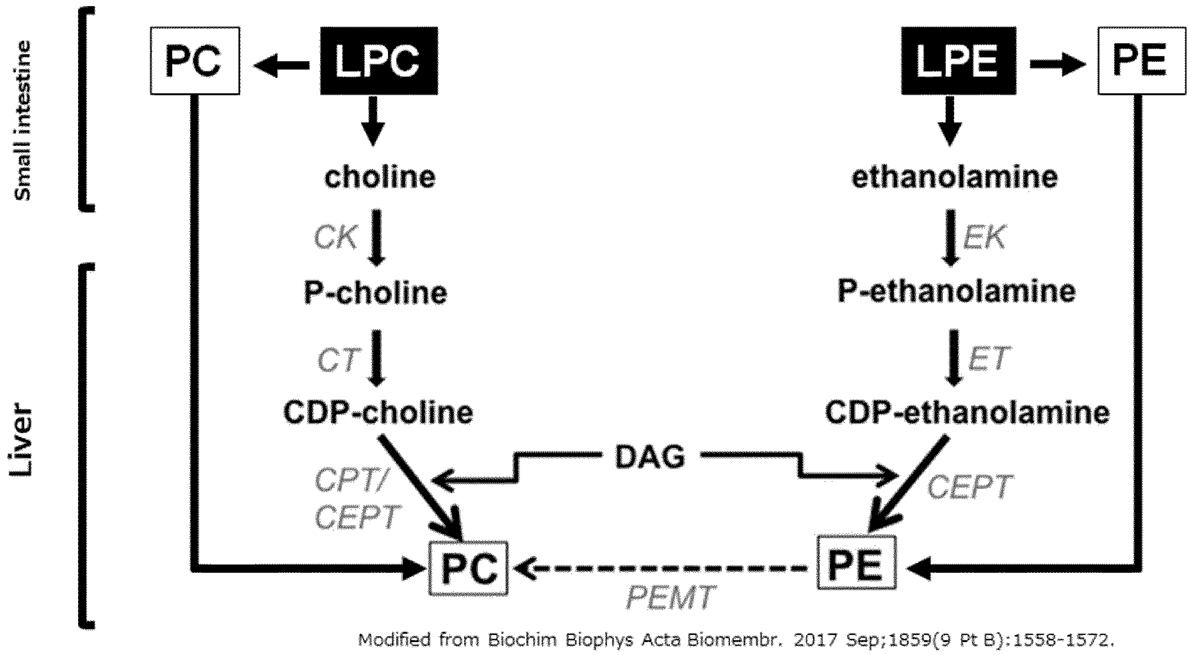

[Figure 3]

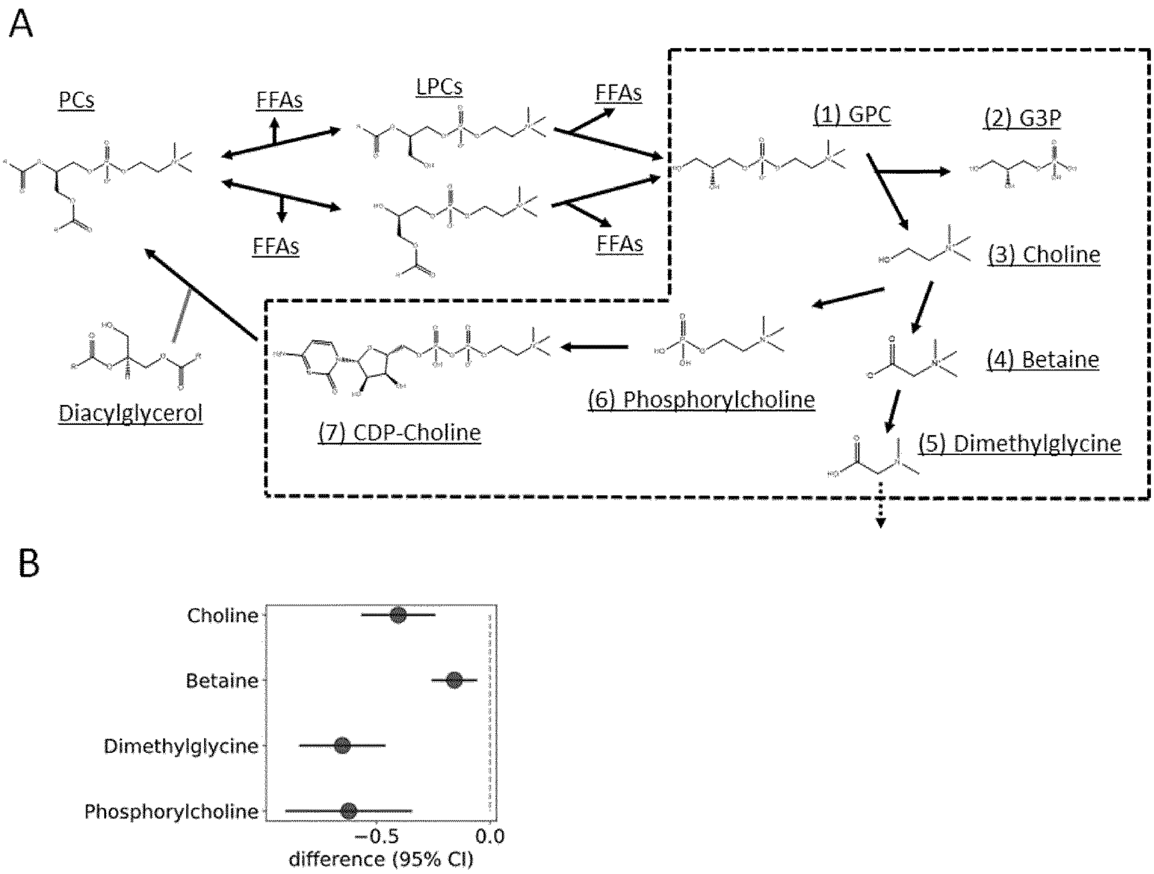


[Figure 4]

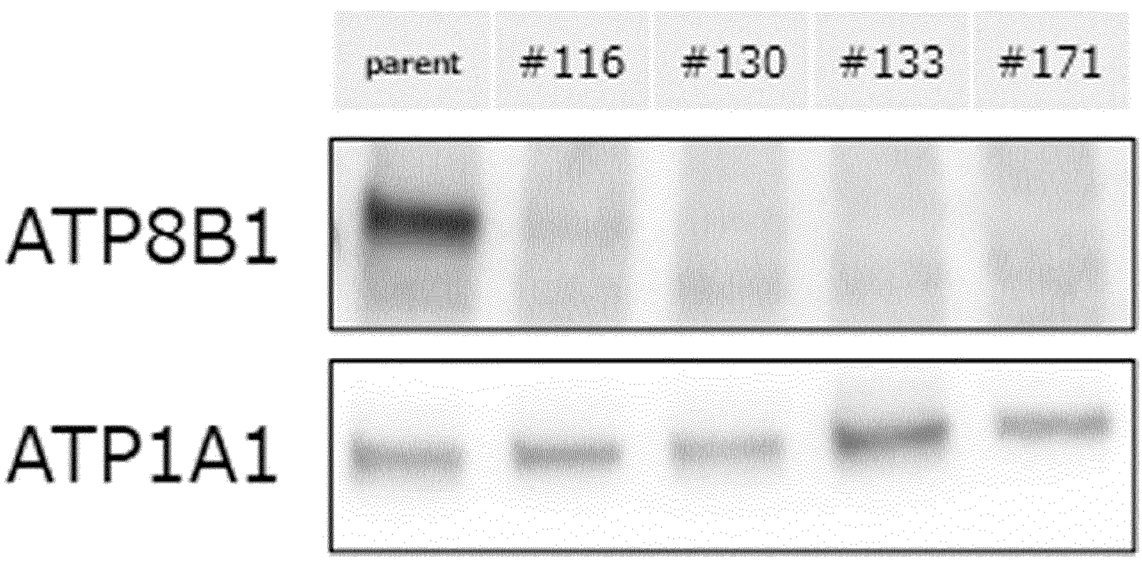

[Figure 5]

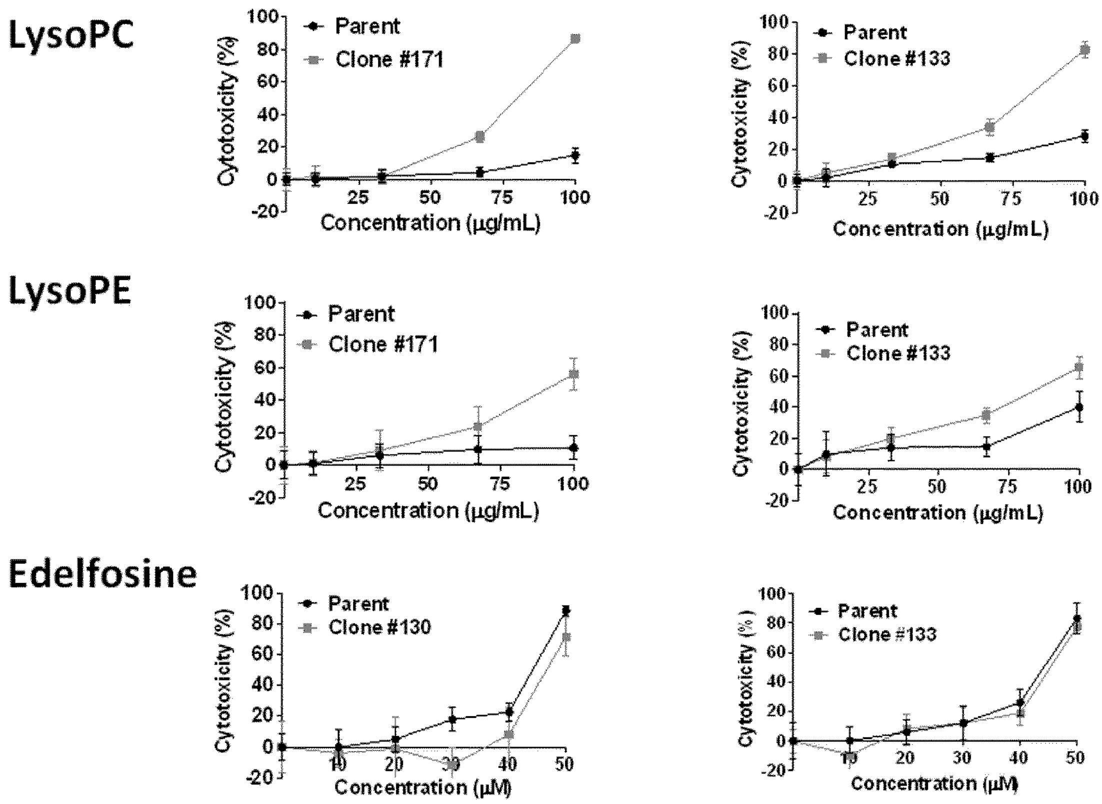


[Figure 6]

18:1-06:0 NBD PC

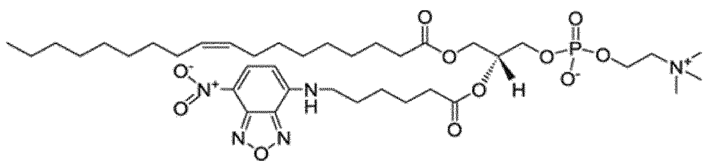

12:0 Lyso NBD PC

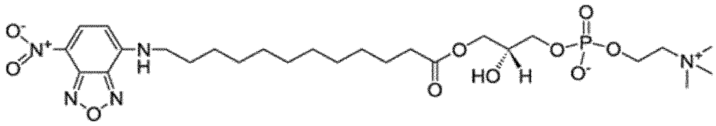

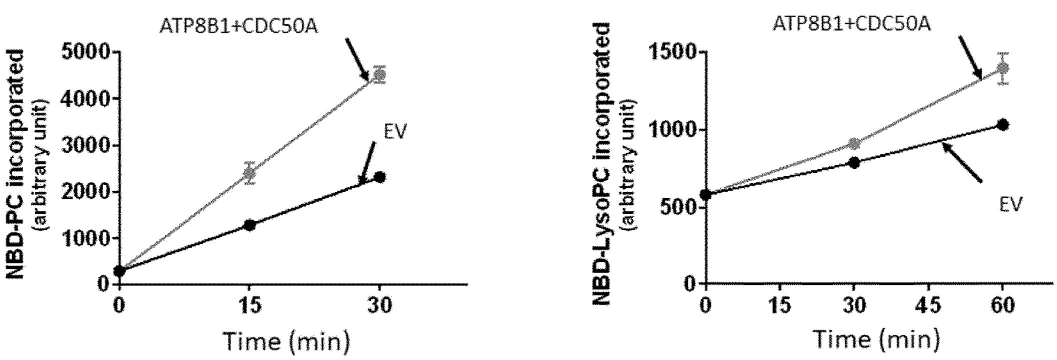

[Figure 7]

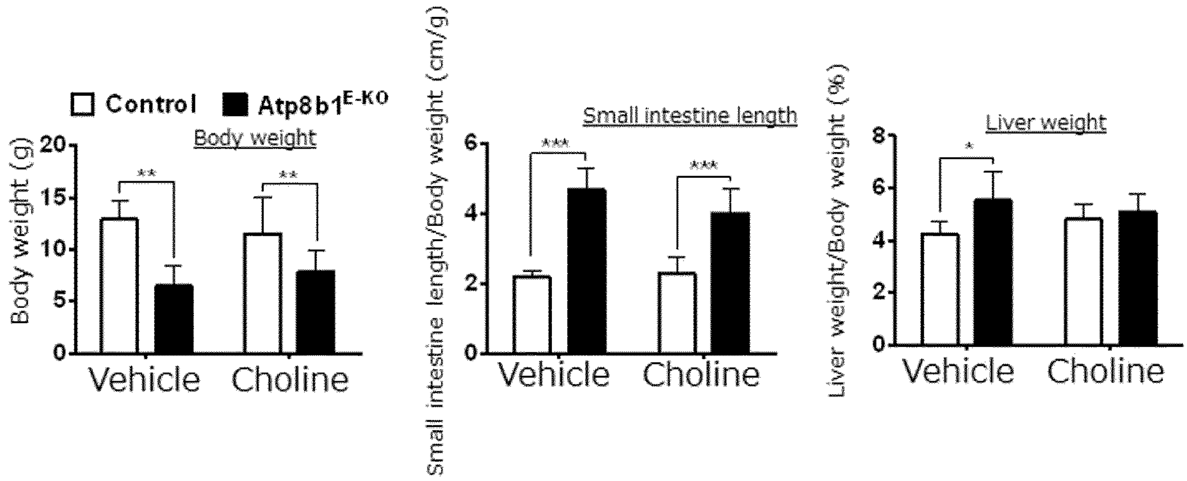


[Figure 8]

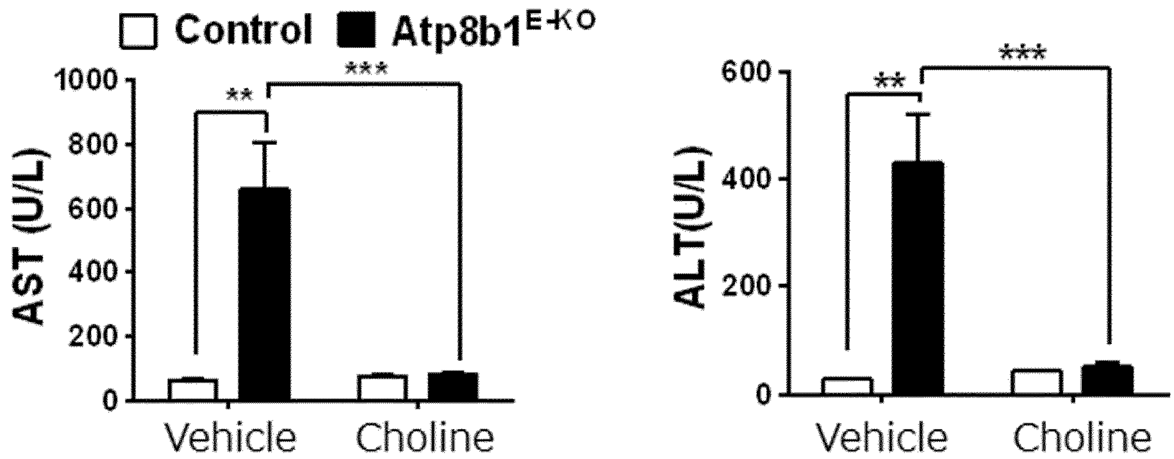

[Figure 9]

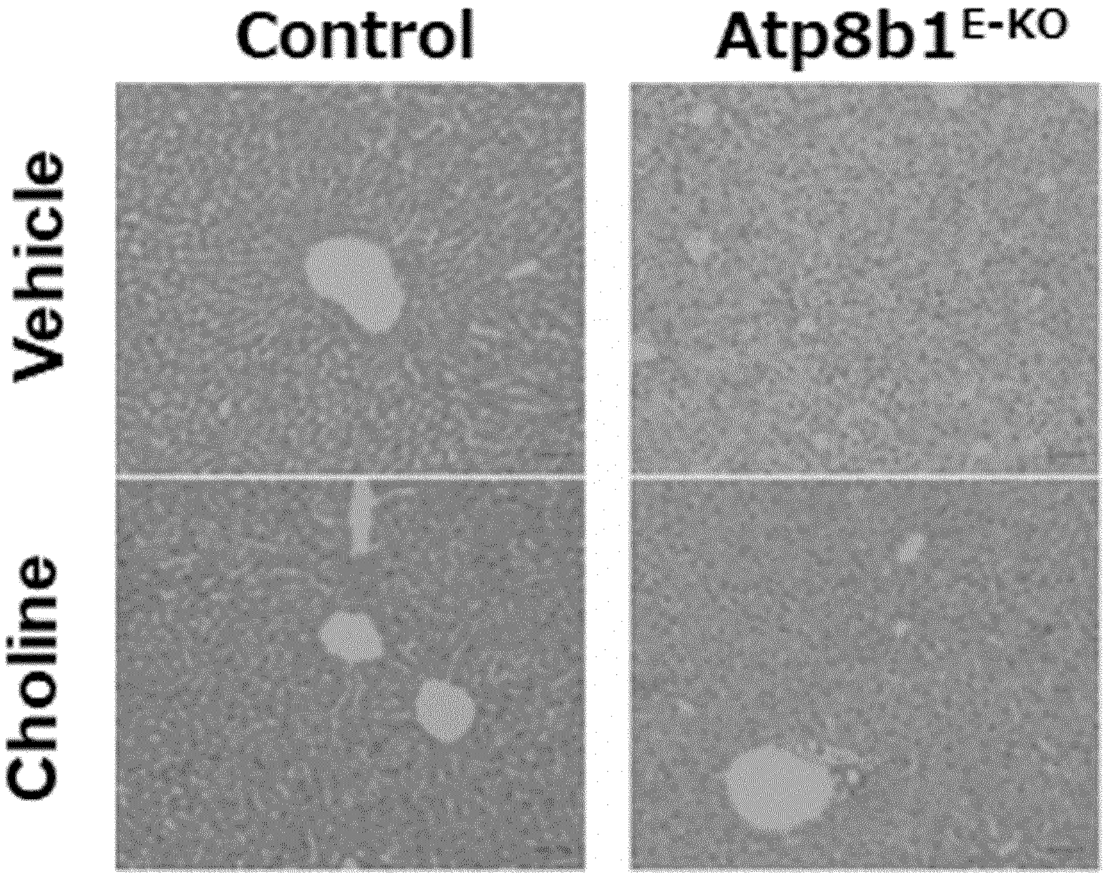

[Figure 10]

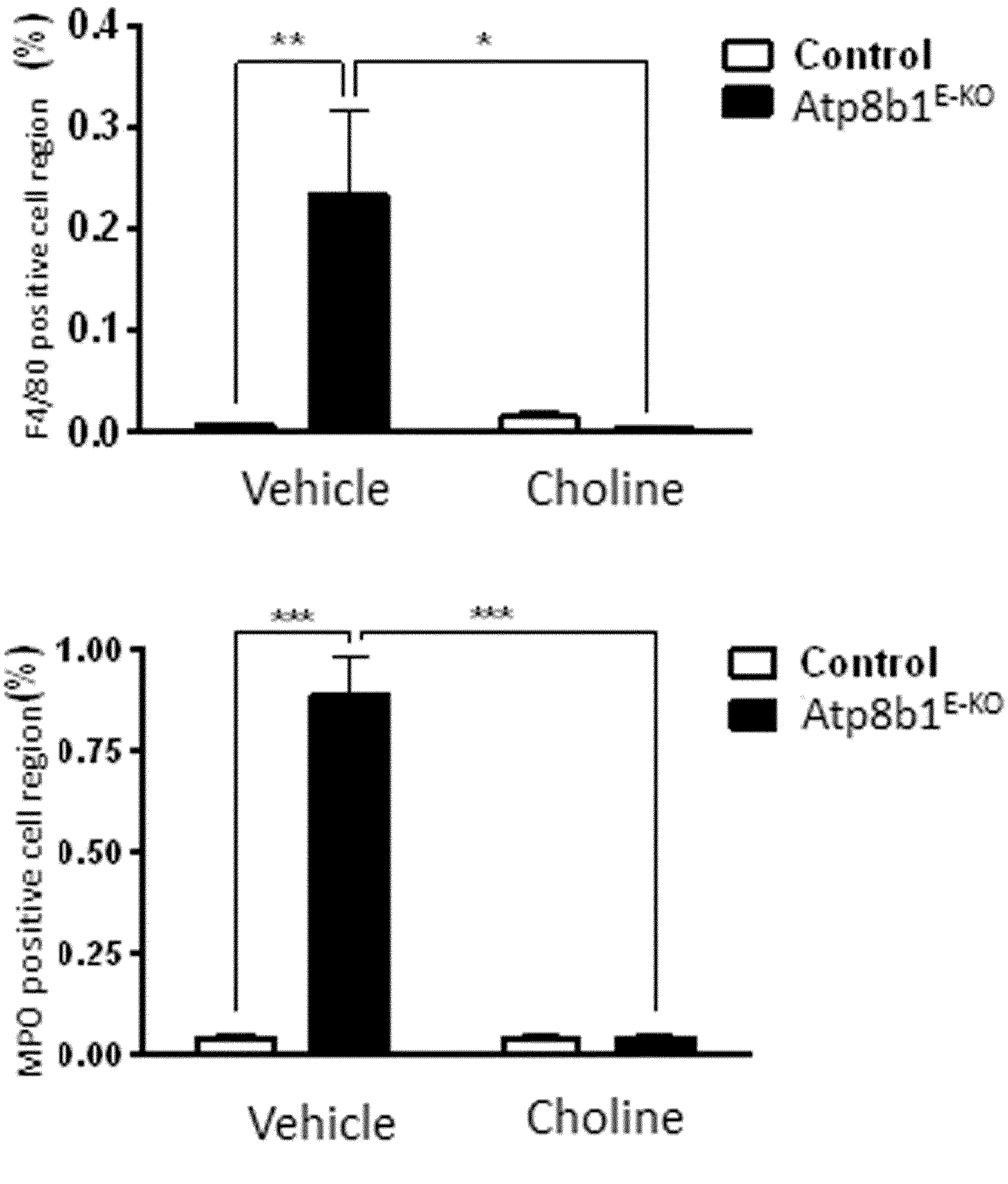
F4/80 positive cell region (%)
0.4
0.3
0.2
0.1
0.0
**
*
Control
Atp8b1E-KO
Vehicle
Choline
MPO positive cell region(%)
1.00
0.75
0.50
0.25
0.00
***
***
Control
Atp8b1E-KO
Vehicle
Choline

[Figure 11]

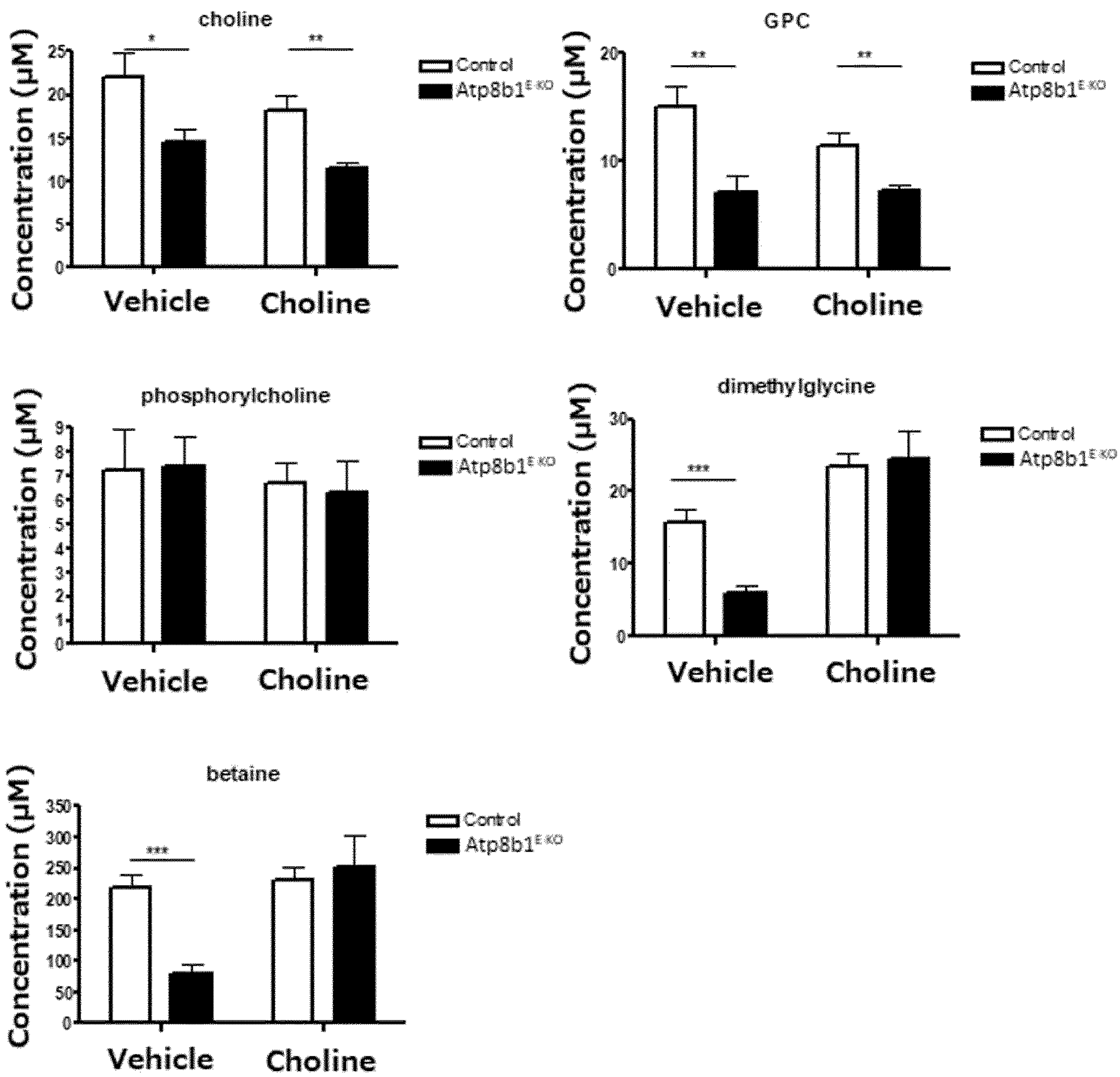
choline
Concentration (μM)
Vehicle
Choline
Control
Atp8b1E-KO
GPC
phosphorylcholine
dimethylglycine
betaine

[Figure 12]

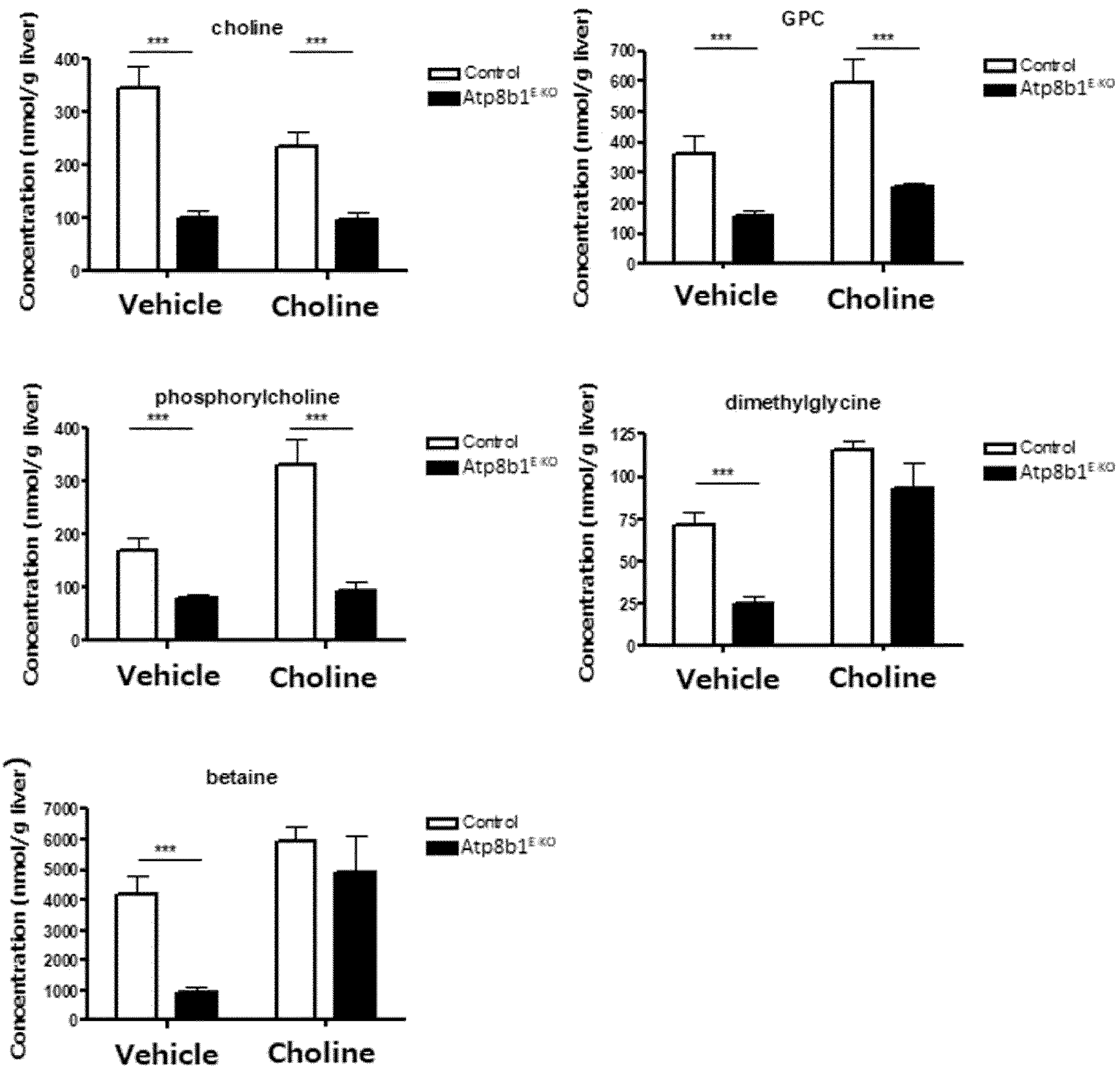
choline
GPC
phosphorylcholine
dimethylglycine
betaine
Concentration (nmol/g liver)
Vehicle
Choline
Control
Atp8b1E-KO
***

[Figure 13]

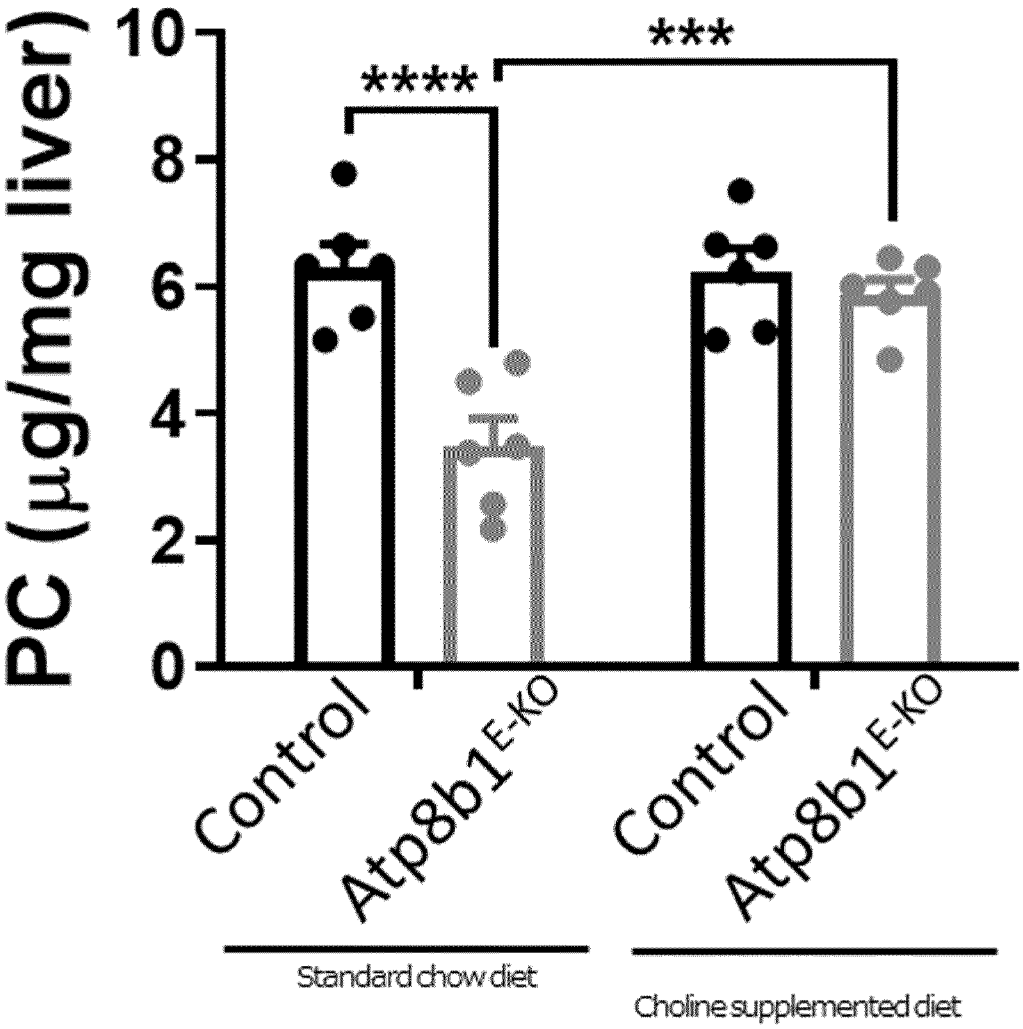
PC (µg/mg liver)
10
8
6
4
2
0
****
***
Control
Atp8b1E-KO
Control
Atp8b1E-KO
Standard chow diet
Choline supplemented diet

[Figure 14]

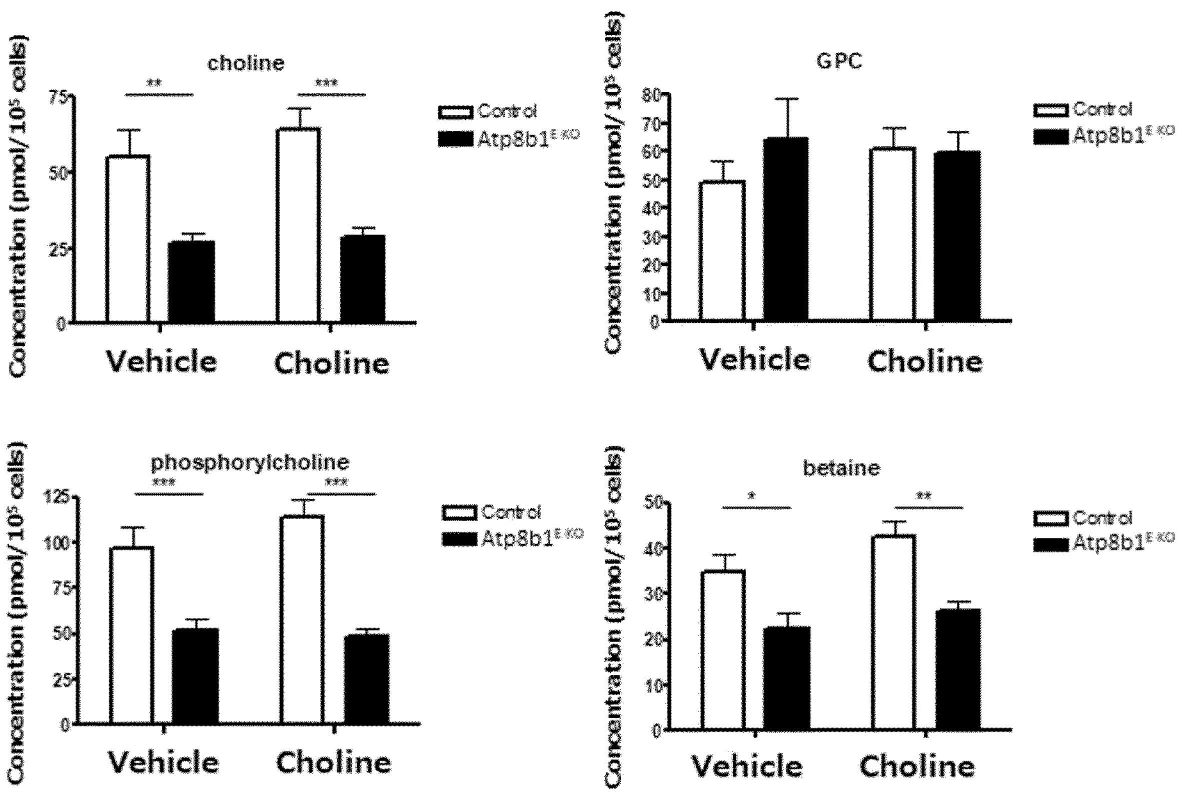
choline
GPC
phosphorylcholine
betaine
Concentration (pmol/10⁵ cells)
Vehicle
Choline
Control
Atp8b1E-KO
**
***
*

[Figure 15]

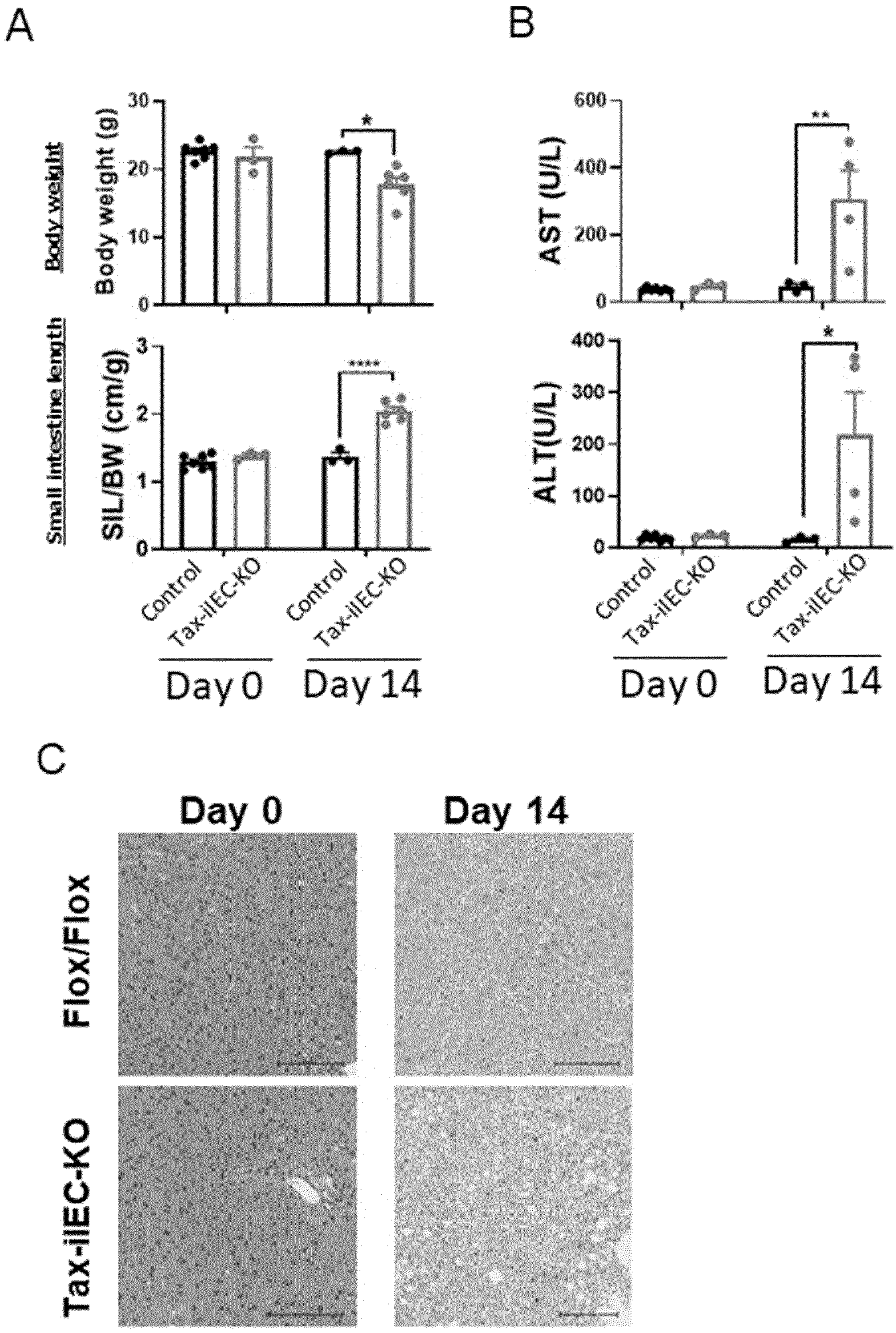
A
Body weight
Body weight (g)
30
20
10
0
*
Small intestine length
SIL/BW (cm/g)
3
2
1
0
****
Control
Tax-iIEC-KO
Control
Tax-iIEC-KO
Day 0
Day 14
B
AST (U/L)
600
400
200
0
**
ALT(U/L)
400
300
200
100
0
*
Control
Tax-iIEC-KO
Control
Tax-iIEC-KO
Day 0
Day 14
C
Day 0
Day 14
Flox/Flox
Tax-iIEC-KO

[Figure 16]

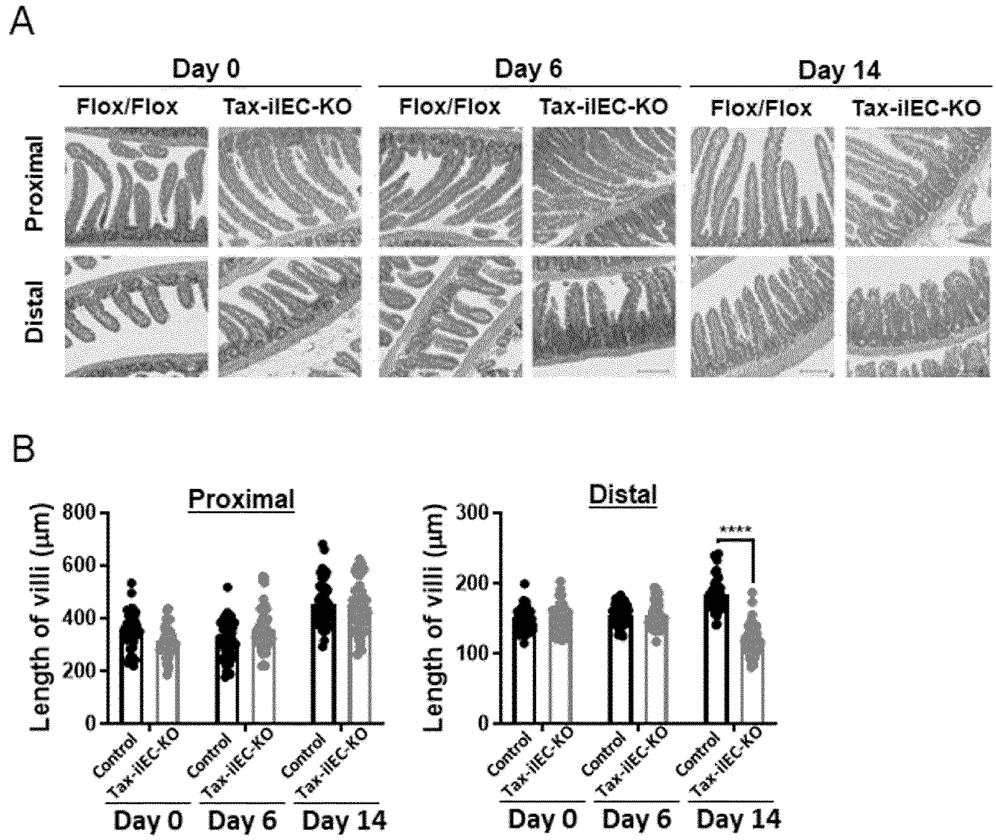


[Figure 17]

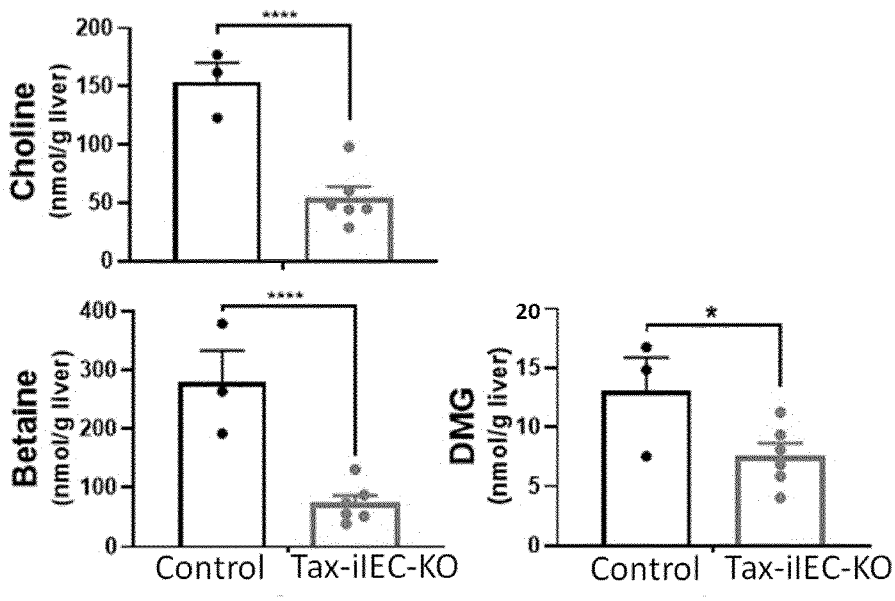

[Figure 18]

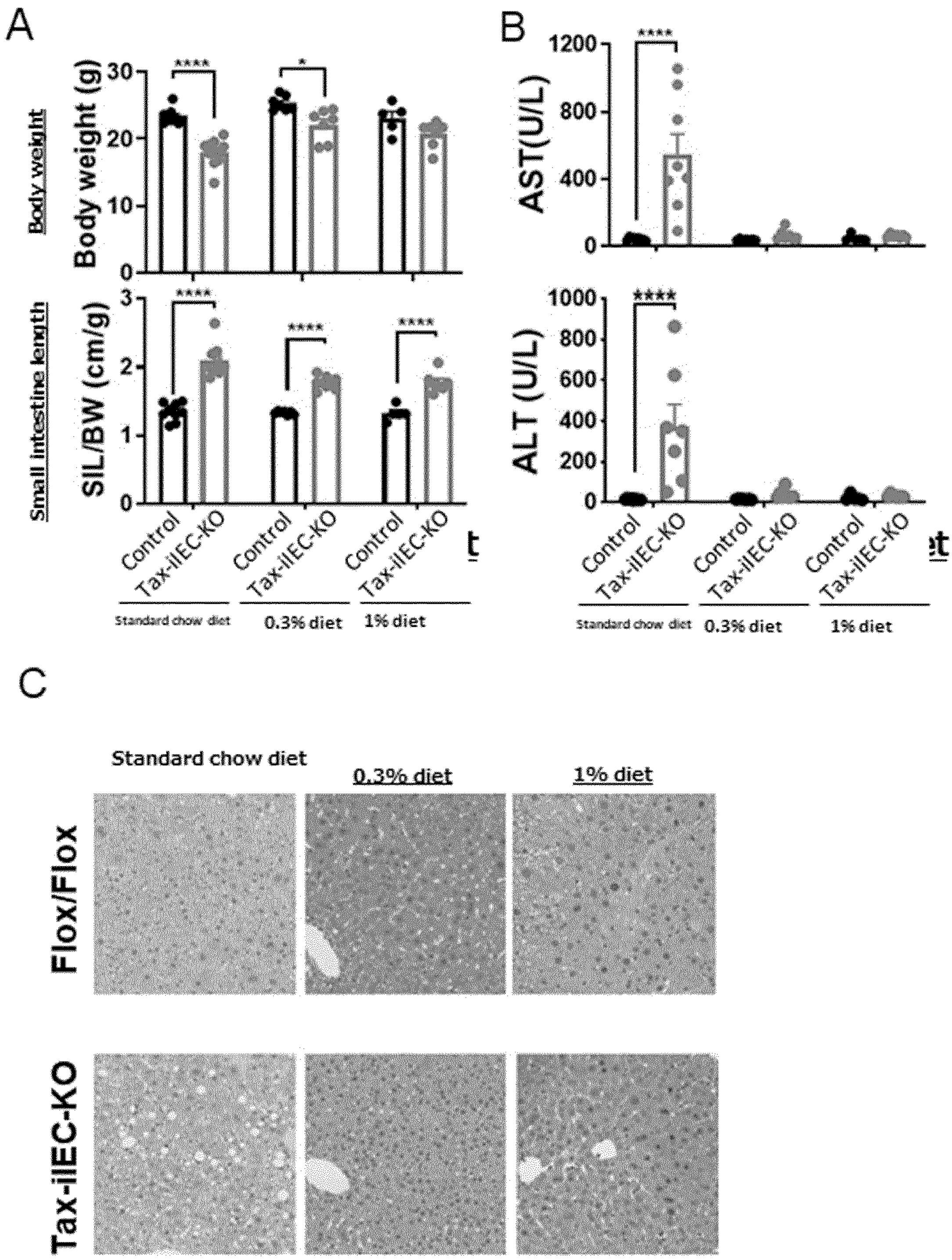
A
Body weight
Body weight (g)
Small intestine length
SIL/BW (cm/g)
B
AST(U/L)
ALT (U/L)
Control
Tax-iIEC-KO
Standard chow diet
0.3% diet
1% diet
C
Standard chow diet
0.3% diet
1% diet
Flox/Flox
Tax-iIEC-KO

[Figure 19]

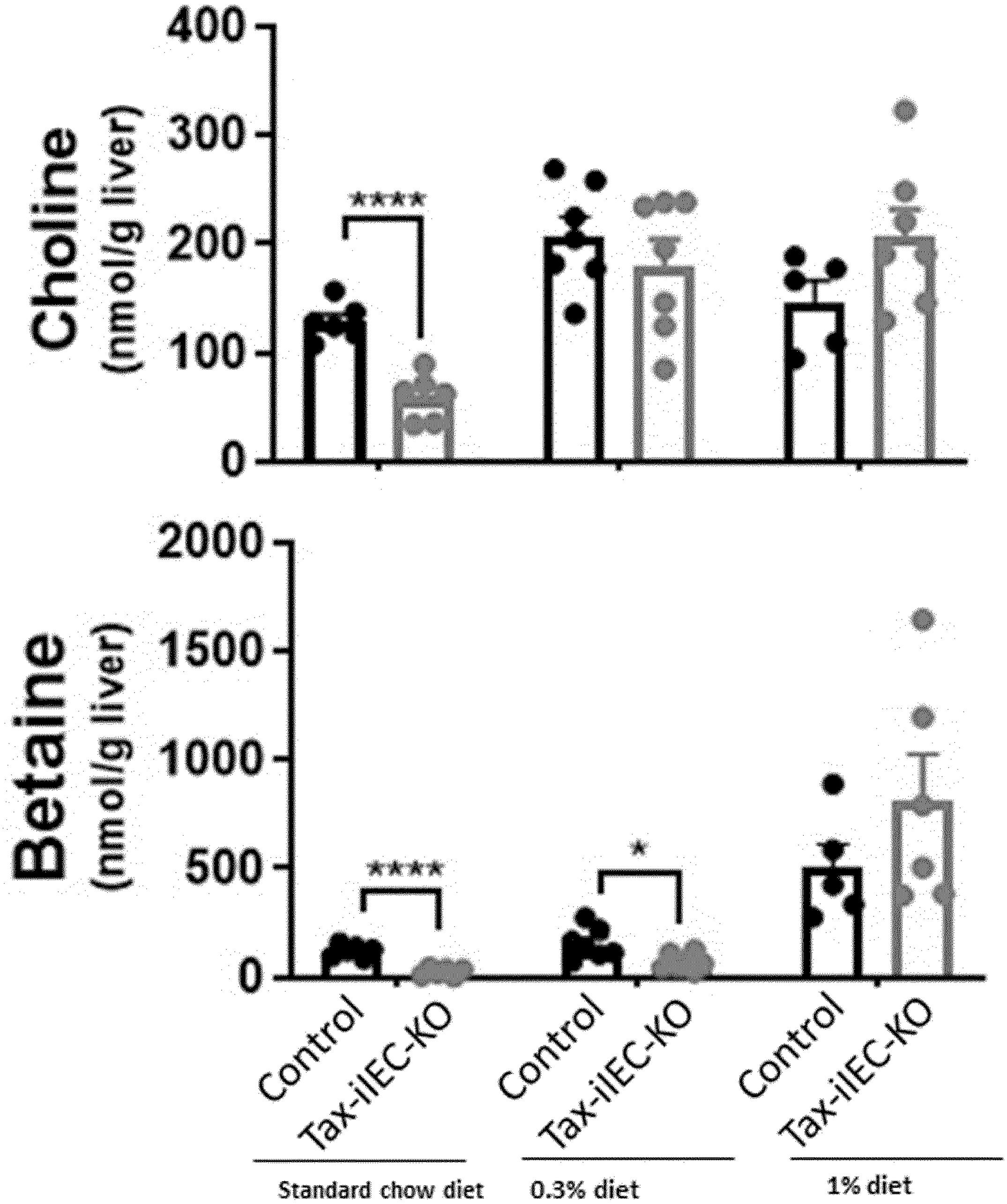
Choline
(nmol/g liver)
400
300
200
100
0
****
Betaine
(nmol/g liver)
2000
1500
1000
500
0
****
*
Control
Tax-iIEC-KO
Control
Tax-iIEC-KO
Control
Tax-iIEC-KO
Standard chow diet
0.3% diet
1% diet

[Figure 20]

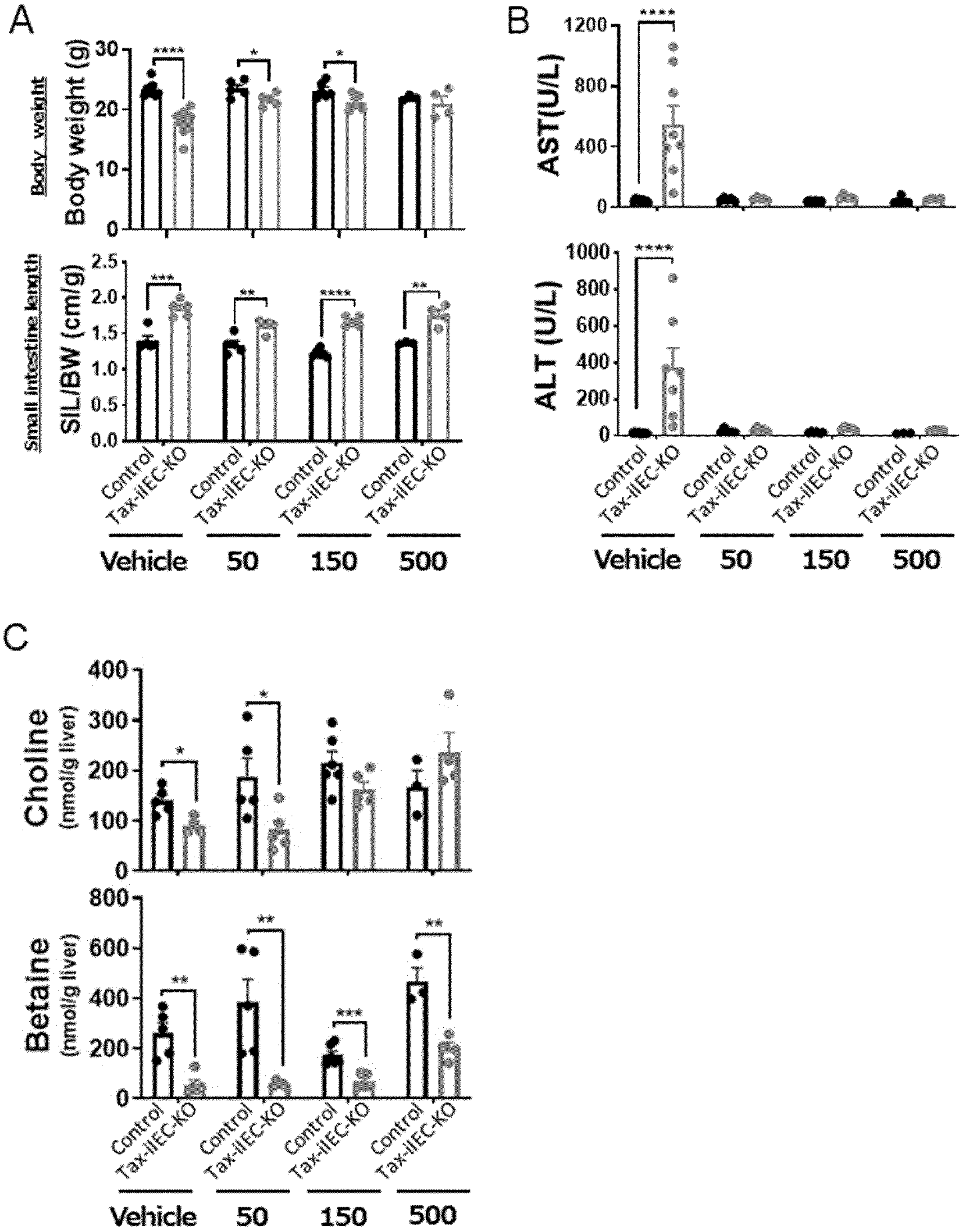
A
Body weight
Body weight (g)
Small intestine length
SIL/BW (cm/g)
B
AST(U/L)
ALT (U/L)
C
Choline
(nmol/g liver)
Betaine
(nmol/g liver)
Control
Tax-iIEC-KO
Vehicle
50
150
500

[Figure 21]

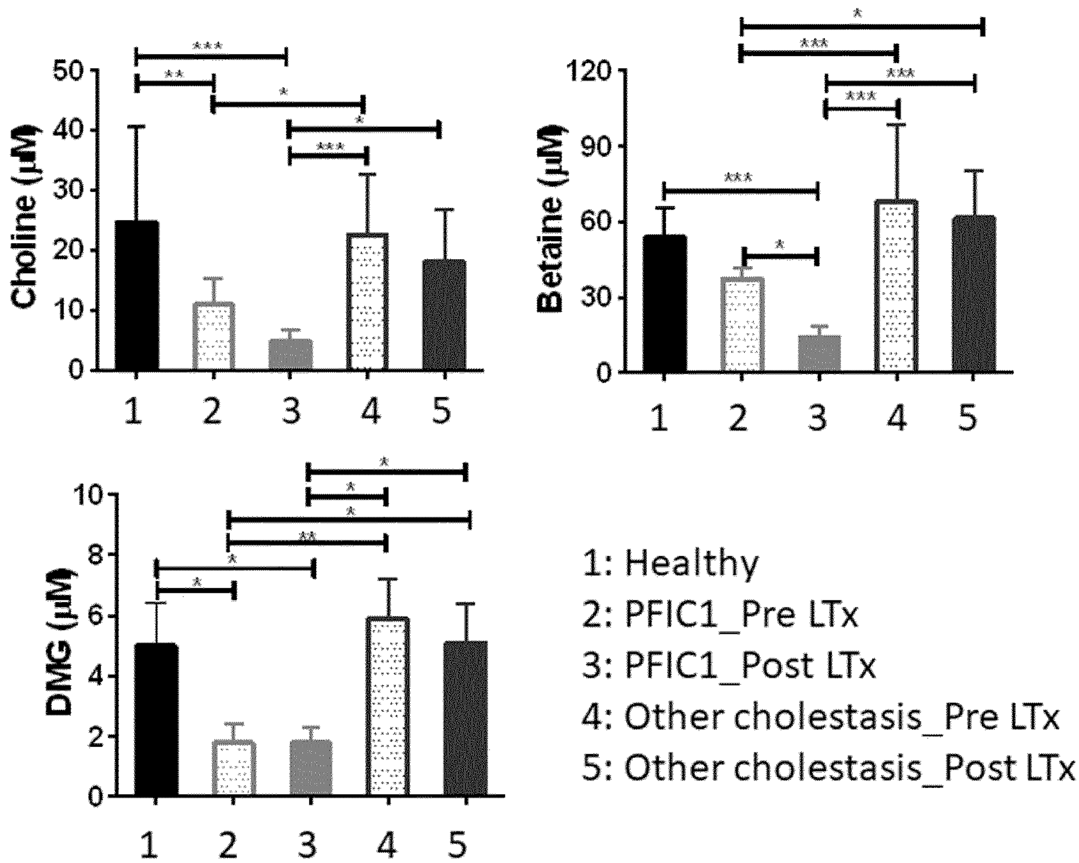
Choline (µM)
Betaine (µM)
DMG (µM)
1: Healthy
2: PFIC1_Pre LTx
3: PFIC1_Post LTx
4: Other cholestasis_Pre LTx
5: Other cholestasis_Post LTx

[Figure 22]

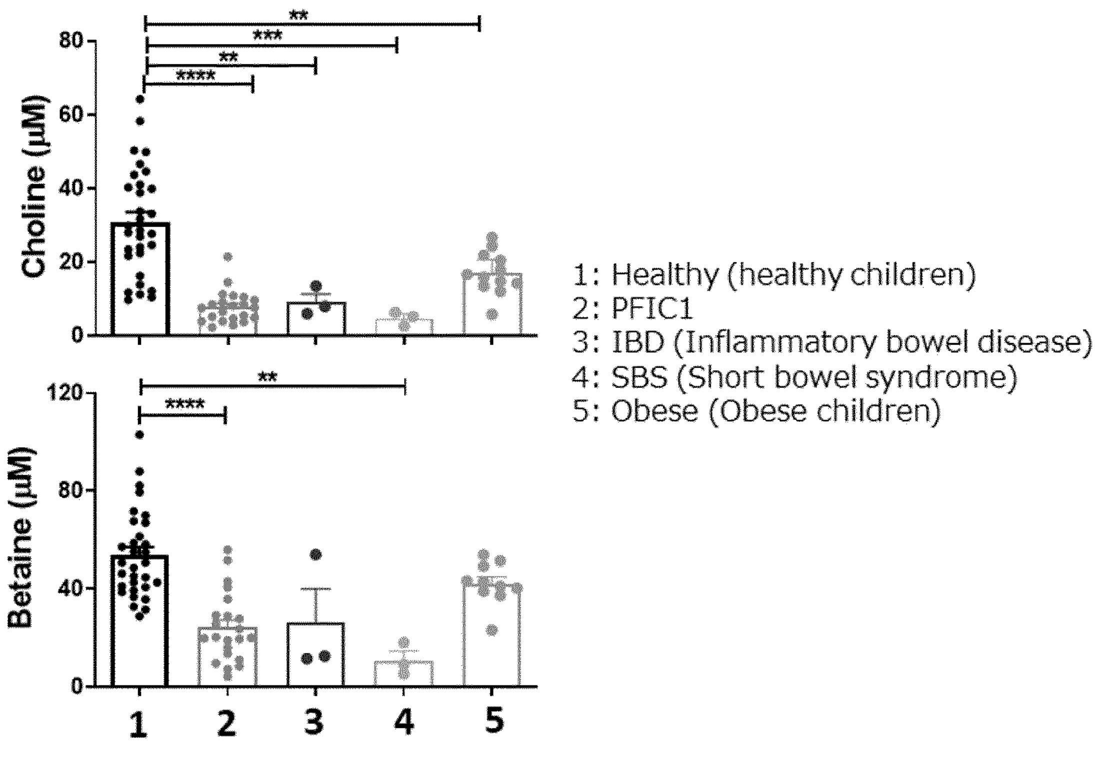
Choline (μM)
80
60
40
20
0
**
***
**
****
Betaine (μM)
120
80
40
0
**
****
1
2
3
4
5
1: Healthy (healthy children)
2: PFIC1
3: IBD (Inflammatory bowel disease)
4: SBS (Short bowel syndrome)
5: Obese (Obese children)

## INTERNATIONAL SEARCH REPORT

International application No.
**PCT/JP2022/044650**

**A. CLASSIFICATION OF SUBJECT MATTER**

***A61P 1/00***(2006.01)i; ***A61P 1/16***(2006.01)i; ***A61P 43/00***(2006.01)i; ***C12N 15/09***(2006.01)i; ***A61K 31/14***(2006.01)i; ***A61K 31/198***(2006.01)i; ***A61K 31/205***(2006.01)i
FI: A61K31/14 ZNA; A61K31/205; A61K31/198; A61P43/00 105; A61P1/00; A61P1/16; C12N15/09 110

According to International Patent Classification (IPC) or to both national classification and IPC

**B. FIELDS SEARCHED**

Minimum documentation searched (classification system followed by classification symbols)

A61P1/00; A61P1/16; A61P43/00; C12N15/09; A61K31/14; A61K31/198; A61K31/205

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | BUCHMAN, ALAN L. et al. Choline Deficiency: A Cause of Hepatic Steatosis During Parenteral Nutrition That Can Be Reversed With Intravenous Choline Supplementation. HEPATOLOGY. 1995, vol. 22, pp. 1399-1403<br>title, abstract, drawings, etc. | 1, 2, 4-6 |
| X | 日比野英彦. コリン関連化合物による脂肪肝治療の可能性. ビタミン. October 2021, vol. 95, no. 10, pp. 441-448, (HIBINO, Hidehiko. Possibility of fatty liver treatment with choline-related compounds. Vitamins.)<br>title, abstract, fig. 2, 3, etc., drawings, p. 441, right column, lines 2-4, p. 444, right column, "Choline, PC, Fatty Liver", p. 445, left column, lines 29-33 | 1-10 |
| Y | | 2, 3, 8, 9 |
| X | WAN, Sereana, et al. Impaired Hepatic Phosphatidylcholine Synthesis Leads to Cholestasis in Mice Challenged With a High-Fat Diet. Hepatol. Commun. 2019, vol. 3, no. 2, pp. 262-276<br>title, abstract, paragraph 1 | 1, 2, 4-8, 10 |
| Y | p. 272, left column, fig. 7 | 2, 3, 8, 9 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 January 2023** | **07 February 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/044650**

**Box No. I** **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

"Annex C/ST.25 text file format" should be read as "ST.26 format".

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SAMBROTTA et al.** *Nat. Genet.,* 2014, vol. 46, 326-328
- **GOMEZ-OSPINA et al.** *Nat. Commun.,* 2014, vol. 7, 10713
- **FOLMER et al.** *Biochim. Biophys. Acta - Mol. Cell Biol. Lipids,* 2009, vol. 1791, 628-635
- **FRANKENBERG et al.** *Hepatology,* 2008, vol. 48, 1896-1905
- **HASEGAWA et al.** *Orphanet J. Rare Dis.,* 2014, vol. 9, 1-9
- *Biochim Biophys Acta Biomembr.,* 2017, vol. 1859, 1558-1575
- **RADETIC et al.** *Dig Dis Sci.,* 25 November 2022
- **ROBERTS et al.** *J Crohns Colitis.,* 07 September 2020, vol. 14 (8), 1119-1148
- **FORSDICK et al.** *J Pediatr Surg.,* December 2019, vol. 54 (12), 2554-2558
- **ZOU et al.** *Inflamm Bowel Dis.,* 18 October 2019, vol. 25 (11), 1764-1772
- *Biochim Biophys Acta Biomembr.,* September 2017, vol. 1859 (9), 1558-1572
- **MIZUNO-IIJIMA et al.** *Methods,* 2021, vol. 191, 23-31
- **YUAN et al.** *Nat. Protoc.,* 2012, vol. 7 (5), 872-881, https://doi.org/10.103 8/nprot.2012.024
- **SATOMI et al.** *J. Chromatogr. B Analyt. Technol. Biomed. Life. Sci.,* 2017, vol. 1063, 93-100, https://doi.org/10.1016/j.jchromb.2017.08.020.
- **SUZUKI et al.** *Nature,* 2010, vol. 468, 834-838
- **TAKATSU et al.** *J. Biol. Chem.,* 2014, vol. 289, 33543-33556